# EUROPÄISCHE PATENTANMELDUNG

(11) **EP 1 905 483 A1**
(43) Veröffentlichungstag der Anmeldung: **02.04.2008**
(21) Anmeldenummer: 06020202.5
(22) Anmeldetag: 27.09.2006
(51) Int. Cl.: A61Q 17/02, A61Q 19/00, A61Q 19/10, A61K 8/04, A61K 8/37

(54) **Alkylbenzoat Gemische**

(71) Anmelder: Cognis IP Management GmbH, 40589 Düsseldorf (DE)
(72) Erfinder: Ansmann, Achim, 40699 Erkrath (DE); Kawa, Rolf, 40789 Monheim (DE); Zander, Lars, 41569 Rommerskirchen (DE); Hans-Dieter, Clages, 40721 Hilden (DE)

(57) **Zusammenfassung**

Gegenstand der Erfindung sind Alkylbenzoat Gemische, wobei die Summe der C12- und C14- Alkylbenzoate größer gleich 85%, bezogen auf die Gesamtsumme der Alkylbenzoate, beträgt sowie die Verwendung dieser Alkylbenzoat Gemische in kosmetischen und/oder pharmazeutischen Zubereitungen, insbesondere als Ölkomponente.

## Beschreibung

### Gebiet der Erfindung

Die Erfindung befindet sich auf dem Gebiet der kosmetischen Inhaltsstoffe und betrifft Alkylbenzoat Gemische definierter Zusammensetzung.

### Stand der Technik

Im Bereich kosmetischer Zubereitungen für die Haut- und Haarpflege werden vom Verbraucher eine Vielzahl von Anforderungen gestellt: Abgesehen von reinigenden und pflegenden Effekten, wird Wert auf so unterschiedliche Parameter wie höchstmögliche dermatologische Verträglichkeit, gute rückfettende Eigenschaften, elegantes Erscheinungsbild, optimaler sensorischer Eindruck und Lagerstabilität gelegt.

Zubereitungen, die zur Reinigung und Pflege der menschlichen Haut und der Haare eingesetzt werden, enthalten in der Regel neben einer Reihe von oberflächenaktiven Substanzen, vor allem Ölkörper und Wasser. Als Ölkörper/Emollients werden beispielsweise Kohlenwasserstoffe, Esteröle sowie pflanzliche und tierische Öte/Fette/Wachse eingesetzt. Um die hohen Anforderungen des Marktes bezüglich sensorischer Eigenschaften und optimaler dermatologischer Verträglichkeit zu erfüllen, werden kontinuierlich neue Ölkörper entwickelt und getestet.

Insbesondere in Zubereitungen, welche UV-Lichtschutzfilter enthalten, ist die Löslichkeit und Stabilität der UV-Lichtschutzfilter in den kosmetischen Zubereitungen und insbesondere in den Ölkörpern entscheidend für die UV-Lichtschutzwirkung des kosmetischen Produktes. Gleichzeitig sollen solche Ölkörper sowohl selbst auch vor allen in den kosmetischen Zubereitungen diesen einen sensorisch leichten Eindruck vermitteln.

Als Ölkörper für kosmetische Zubereitungen sind verschiedene Alkylbenzoate im Stand der Technik bekannt, so z.B. die unter den Handelsnamen Cetiol®AB (Cognis Deutschland GmbH & Co. KG) oder Finsolv®TN (Finetex) erhältlichen C12-C15 Alkylbenzoate oder die unter dem Handelsnamen Finsolv G-2 (Finetex) erhältlichen C16-C17 Alkylbenzoate, ebenso wie die unter dem Handelsnamen Finolv-116 (Finetex) erhältlichen C18 Alkylbenzoate.

Die Alkylbenzoate des Standes der Technik sind jedoch bezüglich der Löslichkeit von UV-Lichtschutzfiltern, insbesondere von kristallinen UV-Lichtschutzfiltern sowie hinsichtlich ihrer Sensorik verbesserungsbedürftig.

Die Aufgabe der vorliegenden Erfindung hat darin bestanden, verbesserte Ölkörper zu Verfügung zu stellen, welche sich insbesondere durch eine verbesserte Löslichkeit für UV-Lichtschutzfilter auszeichnen und gegenüber den bekannten Ölkörper gleichzeitig eine verbesserte Sensorik aufweisen.

Überraschenderweise wurde gefunden, dass Alkybenzoate Gemische mit einer definierten Alkylkettenverteilung die erfindungsgemäße Aufgabe lösen

### Beschreibung der Erfindung

### Alkvlbenzoat Gemische

Der Begriff "Alkylbenzoat" umfasst Ester von Benzoesäure der allgemeinen Formel (I), wobei R für einen aliphatischen, aromatischen, gesättigten, einfach sowie mehrfach ungesättigten, linearen und verzweigten Alkylreste mit C4 bis C22 C-Atomen steht.

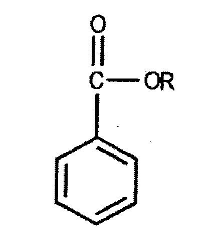

In einer bevorzugten Ausführungsform steht R für einen aliphatischen Alkylrest.

Der Begriff "C12-Alkylbenzoat" beschreibt demnach eine Verbindung gemäß Formel (I), in der R einen linearen oder verzweigten, gesättigten oder ungesättigten Alkylrest mit 12 C Atomen darstellt. Der Begriff "Alkylbenzoat mit einer C-Kettenlänge von 12" wird synonym für solche Verbindungen verwendet. Beide Begriffe umfassen sowohl Einzelverbindungen, wie z.B. Dodecylbenzoat, als auch Mischung verschiedener Alkylbenzoate, bei welchen C = 12 ist, wie z.B. Dodecylbenzoat und 2-Ethyl-1-decylbenzoat.

Gegenstand der Erfindung sind Alkylbenzoat Gemische, dadurch gekennzeichnet, dass der Anteil an ungeradzahligen Alkylbenzoaten kleiner gleich 40%, insbesondere kleiner gleich 30%, bezogen auf die Gesamtsumme der Alkylbenzoate, beträgt. In einer bevorzugten Ausführungsform beträgt der Anteil an ungradzahligen Alkylbenzoaten kleiner gleich 20, insbesondere kleiner gleich 10, kleiner gleich 5, kleiner gleich 3% bezogen auf die Gesamtsumme der Alkylbenzoate.

Der Begriff "Alkylbenzoate Gemische" bezeichnet Mischungen aus Alkylbenzoaten mit verschiedenen C- Kettenlängen. In einer Ausführungsform werden damit Gemische umfasst, welche mindestens 2 Alkylbenzoate unterschiedlicher Alkykettenlänge enthalten, die ausgewählt sind aus der Gruppe der C8 bis C16 Alkylbenzoate. In einer bevorzugten Ausführungsform werden damit Gemische umfasst, welche C12 und/oder C14 Alkylbenzoate enthalten.

Als "ungeradzahlige Alkylbenzoate" werden die Verbindungen der Formel (I) bezeichnet, bei denen R für Alkylrest mit 5, 7, 9, 11, 13, 15, 17, 19 und/oder 21 C-Atomen steht.

Eine Ausführungsform der Erfindung betrifft Alkylbenzoat Gemische, dadurch gekennzeichnet, dass der Anteil an ungeradzahligen Alkylbenzoaten kleiner gleich 50%, insbesondere kleiner gleich 40%, insbesondere kleiner gleich 10, kleiner gleich 5, kleiner gleich 3% bezogen auf die Gesamtsumme der Alkylbenzoate, beträgt und wobei die Summe der Alkylbenzoate mit einer C-Kettenlänge von kleiner gleich 10 kleiner gleich 3%, bezogen auf die Gesamtsumme der Alkylbenzoate, beträgt. Bevorzugt ist die Summe der Alkylbenzoate mit einer C-Kettenlänge von kleiner gleich 10 kleiner gleich 2 %, insbesondere kleiner gleich 1,5 %, insbesondere kleiner gleich 1 % bezogen auf die Gesamtsumme der Alkylbenzoate.

Eine Ausführungsform der Erfindung betrifft Alkylbenzoat Gemische, dadurch gekennzeichnet, dass der Anteil an ungeradzahligen Alkylbenzoaten kleiner gleich 50%, insbesondere kleiner gleich 40%, bevorzugt kleiner gleich 30, insbesondere kleiner gleich 10, kleiner gleich 5, kleiner gleich 3% bezogen auf die Gesamtsumme der Alkylbenzoate, beträgt und wobei die Summe der Alkylbenzoate mit einer C-Kettenlänge von größer 14 kleiner gleich 15% bezogen auf die Gesamtsumme der Alkylbenzoate, beträgt. Bevorzugt ist die Summe der Alkylbenzoate mit einer C-Kettenlänge von größer 14, kleiner gleich 10 %, bevorzugt kleiner gleich 8%, insbesondere kleiner gleich 4%, insbesondere kleiner gleich 2%, bezogen auf die Gesamtsumme der Alkylbenzoate.

Eine Ausführungsform der Erfindung betrifft Alkylbenzoat Gemische, dadurch gekennzeichnet, dass der Anteil an ungeradzahligen Alkylbenzoaten kleiner gleich 50%, insbesondere kleiner gleich 40%, bevorzugt kleiner gleich 30, insbesondere kleiner gleich 10, kleiner gleich 5, kleiner gleich 3% bezogen auf die Gesamtsumme der Alkylbenzoate, beträgt und wobei die Summe der Alkylbenzoate mit einer C-Kettenlänge von größer 14 kleiner gleich 15%, bevorzugt kleiner gleich 10 %, bevorzugt kleiner gleich 8%, insbesondere kleiner gleich 4%, insbesondere kleiner gleich 2%,bezogen auf die Gesamtsumme der Alkylbenzoate, beträgt und die Summe der C12- und C14- Alkylbenzoate größer gleich 85%, bevorzugt größer gleich 90%, insbesondere größer gleich 95%, bezogen auf die Gesamtsumme der Alkylbenzoate, beträgt.

Eine Ausführungsform der Erfindung betrifft Alkylbenzoat Gemische, dadurch gekennzeichnet, dass der Anteil an ungeradzahligen Alkylbenzoaten kleiner gleich 50%, insbesondere kleiner gleich 40%, bevorzugt kleiner gleich 30, insbesondere kleiner gleich 10, kleiner gleich 5, kleiner gleich 3% bezogen auf die Gesamtsumme der Alkylbenzoate, beträgt und wobei die Summe der Alkylbenzoate mit einer C-Kettenlänge von größer 14 kleiner gleich 15%, bevorzugt kleiner gleich 10 %, bevorzugt kleiner gleich 8%, insbesondere kleiner gleich 4%, insbesondere kleiner gleich 2%,bezogen auf die Gesamtsumme der Alkylbenzoate, beträgt und die Summe der Alkylbenzoate mit einer C-Kettenlänge von kleiner gleich 10 kleiner gleich 3%, bevorzugt kleiner gleich 2 %, insbesondere kleiner gleich 1,5 %, insbesondere kleiner gleich 1 % bezogen auf die Gesamtsumme der Alkylbenzoate, beträgt.

Eine Ausführungsform der Erfindung betrifft Alkylbenzoat Gemische, dadurch gekennzeichnet, dass der Anteil an ungeradzahligen Alkylbenzoaten kleiner gleich 50%, insbesondere kleiner gleich 40%, bevorzugt kleiner gleich 30, insbesondere kleiner gleich 10, kleiner gleich 5, kleiner gleich 3% bezogen auf die Gesamtsumme der Alkylbenzoate, beträgt und wobei die Summe der Alkylbenzoate mit einer C-Kettenlänge von größer 14 kleiner gleich 15%, bevorzugt kleiner gleich 10 %, bevorzugt kleiner gleich 8%, insbesondere kleiner gleich 4%, insbesondere kleiner gleich 2%,bezogen auf die Gesamtsumme der Alkylbenzoate, beträgt und die Summe der C12- und C14- Alkylbenzoate größer gleich 85%, bevorzugt größer gleich 90%, insbesondere größer gleich 95%, bezogen auf die Gesamtsumme der Alkylbenzoate, beträgt und die Summe der Alkylbenzoate mit einer C-Kettenlänge von kleiner gleich 10 kleiner gleich 3%, bevorzugt kleiner gleich 2 %, insbesondere kleiner gleich 1,5 %, insbesondere kleiner gleich 1 % bezogen auf die Gesamtsumme der Alkylbenzoate, beträgt.

Gegenstand der Erfindung sind weiterhin Alkylbenzoat Gemische, wobei die Summe der C12- und C14- Alkylbenzoate größer gleich 85%, bezogen auf die Gesamtsumme der Alkylbenzoate, beträgt. Bevorzugt ist die Summe der C12- und C14- Alkylbenzoate größer gleich 90%, insbesondere größer gleich 95%, bezogen auf die Gesamtsumme der Alkylbenzoate.

Der restliche Anteil der Alkylbenzoate besteht üblicherweise Verbindungen der Formel (I), in denen R eine C Zahl zwischen C 4 und C 22, insbesondere zwischen C 6 und C 20 beträgt.

Besonders bevorzugt sind Alkylbenzoat Gemische, wobei die Summe der C12- und C14- Alkylbenzoate größer gleich 85%, bezogen auf die Gesamtsumme der Alkylbenzoate, beträgt und wobei der Anteil an ungeradzahligen Alkylbenzoaten kleiner gleich 50%, insbesondere kleiner gleich 40%, bevorzugt kleiner gleich 30, insbesondere kleiner gleich 10, kleiner gleich 5, kleiner gleich 3% bezogen auf die Gesamtsumme der Alkylbenzoate beträgt.

Bevorzugt ist die Summe der C12- und C14- Alkylbenzoate größer gleich 90%, insbesondere größer gleich 95%, bezogen auf die Gesamtsumme der Alkylbenzoate und der Anteil an verzweigten Alkylbenzoaten kleiner gleich 50%, insbesondere kleiner gleich 40%, bevorzugt kleiner gleich 30 %.

Ein weiterer Gegenstand der vorliegenden Erfindung betrifft ein Alkylbenzoat Gemisch, wobei die Summe der C12- und C14- Alkylbenzoate größer gleich 85%, bezogen auf die Gesamtsumme der Alkylbenzoate, beträgt und wobei die Summe der Alkylbenzoate mit einer C-Kettenlänge von kleiner gleich 10 kleiner gleich 3%, bezogen auf die Gesamtsumme der Alkylbenzoate, beträgt. Bevorzugt ist die Summe der Alkylbenzoate mit einer C-Kettenlänge von kleiner gleich 10 kleiner gleich 2 %, insbesondere kleiner gleich 1,5 %, insbesondere kleiner gleich 1 % bezogen auf die Gesamtsumme der Alkylbenzoate.

Ein weiterer Gegenstand der vorliegenden Erfindung betrifft ein Alkylbenzoat Gemisch, wobei die Summe der C12- und C14- Alkylbenzoate größer gleich 85%, bezogen auf die Gesamtsumme der Alkylbenzoate, beträgt und wobei die Summe der Alkylbenzoate mit einer C-Kettenlänge von kleiner gleich 10 kleiner gleich 3%, bezogen auf die Gesamtsumme der Alkylbenzoate, beträgt. Bevorzugt ist die Summe der Alkylbenzoate mit einer C-Kettenlänge von kleiner gleich 10 kleiner gleich 2 %, insbesondere kleiner gleich 1,5 %, insbesondere kleiner gleich 1 % bezogen auf die Gesamtsumme der Alkylbenzoate, und wobei der Anteil an ungeradzahligen Alkylbenzoaten kleiner gleich 50%, insbesondere kleiner gleich 40%, bevorzugt kleiner gleich 30, insbesondere kleiner gleich 10, kleiner gleich 5, kleiner gleich 3% bezogen auf die Gesamtsumme der Alkylbenzoate beträgt.

Ein weiterer Gegenstand der vorliegenden Erfindung betrifft Alkylbenzoat Gemische, enthaltend C12 und/oder C14 Alkylbenzoate, wobei die Summe der Alkylbenzoate mit einer C-Kettenlänge von größer 14 kleiner gleich 15% bezogen auf die Gesamtsumme der Alkylbenzoate, beträgt. Bevorzugt ist die Summe der Alkylbenzoate mit einer C-Kettenlänge von größer 14, kleiner gleich 10 %, bevorzugt kleiner gleich 8%, insbesondere kleiner gleich 4%, insbesondere kleiner gleich 2%, bezogen auf die Gesamtsumme der Alkylbenzoate.

Bevorzugt sind Alkylbenzoat Gemische, enthaltend C12 und/oder C14 Alkylbenzoate, wobei die Summe der Alkylbenzoate mit einer C-Kettenlänge von größer 14 kleiner gleich 15, bevorzugt kleiner gleich 10%, bevorzugt kleiner gleich 8%, insbesondere kleiner gleich 4%, insbesondere kleiner gleich 2%, bezogen auf die Gesamtsumme der Alkylbenzoate, beträgt, und wobei die Summe der Alkylbenzoate mit einer C-Kettenlänge von kleiner gleich 10 kleiner gleich 3%, bevorzugt kleiner gleich 2 %, insbesondere kleiner gleich 1,5 %, insbesondere kleiner gleich 1 % bezogen auf die Gesamtsumme der Alkylbenzoate beträgt.

Eine bevorzugte Ausführungsform der Erfindung betrifft Alkylbenzoat Gemische, wobei die Summe der C12- und C14- Alkylbenzoate größer gleich 85%, bezogen auf die Alkylbenzoate, beträgt und die Summe der Alkylbenzoate mit einer C-Kettenlänge von größer 14, kleiner gleich 15%, bezogen auf die Gesamtsumme der Alkylbenzoate, beträgt.

Eine bevorzugte Ausführungsform der Erfindung betrifft Alkylbenzoat Gemische, wobei die Summe der C12- und C14- Alkylbenzoate größer gleich 85%, bezogen auf die Gesamtsumme der Alkylbenzoate , beträgt und die Summe der Alkylbenzoate mit einer C-Kettenlänge von kleiner gleich 10 kleiner gleich 15% beträgt und die Summe Alkylbenzoate mit einer C-Kettenlänge von größer 14, kleiner gleich 15%, bezogen auf die Gesamtsumme der Alkylbenzoate, beträgt.

Bei allen diesen Ausführungsformen beträgt die Summe der C12- und C14- Alkylbenzoate jeweils bevorzugt größer gleich 90%, insbesondere größer gleich 95%, bezogen auf die Gesamtsumme der Alkylbenzoate.

Bei allen diesen Ausführungsformen beträgt die Summe der Alkylbenzoate mit einer C-Kettenlänge von kleiner gleich 10 jeweils bevorzugt kleiner gleich 2 %, insbesondere kleiner gleich 1,5 %, insbesondere kleiner gleich 1 % bezogen auf die Gesamtsumme der Alkylbenzoate.

Bei allen diesen Ausführungsformen beträgt die Summe der Alkylbenzoate mit einer C-Kettenlänge von größer 14 jeweils bevorzugt kleiner gleich 10 %, bevorzugt kleiner gleich 8%, insbesondere kleiner gleich 4%, insbesondere kleiner gleich 2%, bezogen auf die Gesamtsumme der Alkylbenzoate.

Ein weiterer Gegenstand der Erfindung betrifft Alkylbenzoat Gemische, wobei die Alkylbenzoate folgende C-Kettenverteilung aufweisen:
(a) C12 Alkylbenzoate größer gleich 60% und
(b) C14 Alkylbenzoate zwischen 15% und 40% bezogen auf die Gesamtsumme der Alkylbenzoate.

In einer besonders bevorzugten Ausführungsform dieser Erfindung, ist die Summe der C12-Alkylbenzoate größer gleich 65%, insbesondere größer gleich 70%, bezogen auf die Gesamtsumme der Alkylbenzoate.

In einer besonders bevorzugten Ausführungsform dieser Erfindung, ist die Summe der C14-Alkylbenzoate zwischen 20% und 35%, bevorzugt zwischen 25% und 30% bezogen auf die Gesamtsummer der Alkylbenzoate.

Ein weiterer Gegenstand der Erfindung betrifft Alkylbenzoat Gemische, wobei die Alkylbenzoate folgende C-Kettenverteilung aufweisen:
(a) C12 Alkylbenzoate größer gleich 60% und
(b) C14 Alkylbenzoate zwischen 15% und 40% bezogen auf die Gesamtsumme der Alkylbenzoate, und der Anteil an ungeradzahligen Alkylbenzoaten kleiner gleich 50%, insbesondere kleiner gleich 40%, bevorzugt kleiner gleich 30, insbesondere kleiner gleich 10, kleiner gleich 5, kleiner gleich 3% bezogen auf die Gesamtsumme der Alkylbenzoate, beträgt.

Eine bevorzugte Ausführungsform der Erfindung betrifft Alkylbenzoat Gemische, wobei die Alkylbenzoate folgende C-Kettenverteilung aufweisen:
(a) C12 Alkylbenzoate größer gleich 60% und (b) C-14 Alkylbenzoate zwischen 15% und 40% und die Summe der Alkylbenzoate mit einer C-Kettenlänge von kleiner gleich 10, ist kleiner gleich 3%, insbesondere kleiner gleich 2%, bevorzugt kleiner gleich 1 %,- jeweils bezogen auf die Gesamtsumme der Alkylbenzoate. In einer besonders bevorzugten Ausführungsform dieser Erfindung, ist die Summe der C12-Alkylbenzoate größer gleich 65%, insbesondere größer gleich 70%, bezogen auf die Gesamtsumme der Alkylbenzoate. In einer besonders bevorzugten Ausführungsform dieser Erfindung, ist die Summe der C14-Alkylbenzoate zwischen 20% und 35%, bevorzugt zwischen 25% und 30%, bezogen auf die Gesamtsumme der Alkylbenzoate.

Eine bevorzugte Ausführungsform der Erfindung betrifft Alkylbenzoat Gemische, wobei die Alkylbenzoate folgende C-Kettenverteilung aufweisen:
(a) C12 Alkylbenzoate größer gleich 60% und (b) C-14 Alkylbenzoate zwischen 15% und 40% und die Summe der Alkylbenzoate mit einer C-Kettenlänge von kleiner gleich 10, ist kleiner gleich 3%, insbesondere kleiner gleich 2%, bevorzugt kleiner gleich 1 %,- jeweils bezogen auf die Gesamtsumme der Alkylbenzoate, und der Anteil an ungradzahligen Alkylbenzoaten kleiner gleich 50%, insbesondere kleiner gleich 40%, bevorzugt kleiner gleich 30, insbesondere kleiner gleich 10, kleiner gleich 5, kleiner gleich 3% bezogen auf die Gesamtsumme der Alkylbenzoate, beträgt. In einer besonders bevorzugten Ausführungsform dieser Erfindung, ist die Summe der C12-Alkylbenzoate größer gleich 65%, insbesondere größer gleich 70%, bezogen auf die Gesamtsumme der Alkylbenzoate. In einer besonders bevorzugten Ausführungsform dieser Erfindung, ist die Summe der C14-Alkylbenzoate zwischen 20% und 35%, bevorzugt zwischen 25% und 30%, bezogen auf die Gesamtsumme der Alkylbenzoate

Eine bevorzugte Ausführungsform der Erfindung betrifft Alkylbenzoat Gemische, wobei die Alkylbenzoate folgende C-Kettenverteilung aufweisen:
(a) C12-Alkylbenzoate größer gleich 60% und (b) C-14-Alkylbenzoate zwischen 15% und 40% und die Summe der Alkylbenzoate mit einer C-Kettenlänge von größer 14, ist kleiner gleich 15%, insbesondere in kleiner gleich 10%, bevorzugt kleiner gleich 8%, insbesondere kleiner gleich 4%, insbesondere kleiner gleich 2 %,- jeweils bezogen auf die Gesamtsumme der Alkylbenzoate. In einer besonders bevorzugten Ausführungsform dieser Erfindung, ist die Summe der C12-Alkylbenzoate größer gleich 65%, insbesondere größer gleich 70%, bezogen auf die Gesamtsumme der Alkylbenzoate. In einer besonders bevorzugten Ausführungsform dieser Erfindung, ist die Summe der C14-Alkylbenzoate zwischen 20% und 35%, bevorzugt zwischen 25% und 30%, bezogen auf die Gesamtsumme der Alkylbenzoate.

Eine bevorzugte Ausführungsform der Erfindung betrifft Alkylbenzoat Gemische, wobei die Alkylbenzoate folgende C-Kettenverteilung aufweisen:
(a) C12-Alkylbenzoate größer gleich 60% und (b) C-14-Alkylbenzoate zwischen 15% und 40% und die Summe der Alkylbenzoate mit einer C-Kettenlänge von größer 14, ist kleiner gleich 15%, insbesondere in kleiner gleich 10%, bevorzugt kleiner gleich 8%, insbesondere kleiner gleich 4%, insbesondere kleiner gleich 2 %,- jeweils bezogen auf die Gesamtsumme der Alkylbenzoate und der Anteil an ungeradzahligen Alkylbenzoaten ist kleiner gleich 50%, insbesondere kleiner gleich 40%, bevorzugt kleiner gleich 30, insbesondere kleiner gleich 10, kleiner gleich 5, kleiner gleich 3% bezogen auf die Gesamtsumme der Alkylbenzoate. In einer besonders bevorzugten Ausführungsform dieser Erfindung, ist die Summe der C12-Alkylbenzoate größer gleich 65%, insbesondere größer gleich 70%, bezogen auf die Gesamtsumme der Alkylbenzoate. In einer besonders bevorzugten Ausführungsform dieser Erfindung, ist die Summe der C14-Alkylbenzoate zwischen 20% und 35%, bevorzugt zwischen 25% und 30%, bezogen auf die Gesamtsumme der Alkylbenzoate.

Eine besonders bevorzugte Ausführungsform der Erfindung betrifft Alkylbenzoat Gemische, wobei die Alkylbenzoate folgende C-Kettenverteilung aufweisen:
(a) C12-Alkylbenzoate größer gleich 60% und (b) C-14-Alkylbenzoate zwischen 15% und 40% und die Summe der Alkylbenzoate mit einer C-Kettenlänge von kleiner gleich 10 ist kleiner gleich 3%, insbesondere kleiner gleich 2%, bevorzugt kleiner gleich 1%, beträgt und die Summe Alkylbenzoate mit einer C-Kettenlänge von größer 14 ist kleiner gleich 15%, insbesondere in kleiner gleich 10%, bevorzugt kleiner gleich 8%, insbesondere kleiner gleich 4%, bevorzugt kleiner gleich 2%,- jeweils bezogen auf die Gesamtsumme der Alkylbenzoate.. In einer besonders bevorzugten Ausführungsform dieser Erfindung, ist die Summe der C12-Alkylbenzoate größer gleich 65%, insbesondere größer gleich 70%, bezogen auf die Gesamtsumme der Alkylbenzoate. In einer besonders bevorzugten Ausführungsform dieser Erfindung, ist die Summe der C14-Alkylbenzoate zwischen 20% und 35%, bevorzugt zwischen 25% und 30%, bezogen auf die Gesamtsumme der Alkylbenzoate.

Eine besonders bevorzugte Ausführungsform der Erfindung betrifft Alkylbenzoat Gemische, wobei die Alkylbenzoate folgende C-Kettenverteilung aufweisen:
(a) C12-Alkylbenzoate größer gleich 60% und (b) C-14-Alkylbenzoate zwischen 15% und 40% und die Summe der Alkylbenzoate mit einer C-Kettenlänge von kleiner gleich 10 ist kleiner gleich 3%, insbesondere kleiner gleich 2%, bevorzugt kleiner gleich 1%, beträgt und die Summe Alkylbenzoate mit einer C-Kettenlänge von größer 14 ist kleiner gleich 15%, insbesondere in kleiner gleich 10%, bevorzugt kleiner gleich 8%, insbesondere kleiner gleich 4%, bevorzugt kleiner gleich 2%,- jeweils bezogen auf die Gesamtsumme der Alkylbenzoate und der Anteil an ungradzahligen Alkylbenzoaten ist kleiner gleich 50%, insbesondere kleiner gleich 40%, bevorzugt kleiner gleich 30, insbesondere kleiner gleich 10, kleiner gleich 5, kleiner gleich 3% bezogen bezogen auf die Gesamtsumme der Alkylbenzoate. In einer besonders bevorzugten Ausführungsform dieser Erfindung, ist die Summe der C12-Alkylbenzoate größer gleich 65%, insbesondere größer gleich 70%, bezogen auf die Gesamtsumme der Alkylbenzoate. In einer besonders bevorzugten Ausführungsform dieser Erfindung, ist die Summe der C14-Alkylbenzoate zwischen 20% und 35%, bevorzugt zwischen 25% und 30%, bezogen auf die Gesamtsumme der Alkylbenzoate.

Eine weitere Ausführungsform der vorliegenden Erfindung betrifft jegliches bisher genanntes Alkylbenzoat Gemisch, wobei die Summe der verzweigten Alkylbenzoate kleiner gleich 10 %, bevorzugt kleiner gleich 5 %, bezogen auf die Gesamtsumme der Alkylbenzoate, beträgt.

Keines der bekannten Alkylbenzoat Gemische des Standes der Technik weist die erfindungsgemäßen Verteilungen auf. Überraschenderweise wurde gefunden, dass Gemische mit der erfindungsgemäßen C-Kettenverteilung nicht nur hoch spreitende Ölkörper sind, sondern diese gleichzeitig ein gegenüber den bekannten Alkylbenzoaten verbessertes Lösevermögen für UV-Lichtschutzfilter aufweisen.

### Herstellung

Die Herstellung der Alkylbenzoate erfolgt nach dem Fachmann bekannten Verfahren. Die Alkylbenzoate können beispielsweise durch Umsetzung von Benzoesäure mit dem entsprechenden Alkohol oder Alkoholgemisch in Gegenwart eines Katalysators erhalten werden. Ein solches Verfahren ist beispielsweise in WO 2004/099117 beschrieben.

Ebenso können die Alkylbenzoate durch Umsetzung von Methylestern der Benzoesäure mit einen Alkoholgemisch erhalten werden, wobei das Alkoholgemisch die erfindungsgemäße C-Verteilung aufweist, die sich dann im Alkylbenzoat Gemisch wieder findet.

Die erfindungsgemäßen Alkylbenzoat Gemische können erhalten werden, in dem man bei der Umsetzung Alkoholgemische einsetzt, welche die erfindungsgemäße C-Verteilung aufweist. Ebenso können die erfindungsgemäßen Alkylbenzoat Gemische erhalten werden durch Mischen der entsprechenden einzelnen Alkylbenzoate.

Der Geruch der durch Veresterung oder Umesterung erhaltenen Produkte kann je nach Bedarf durch Desodorieren verbessert werden, ebenso kann die Farbe gegebenenfalls durch Behandlung mit dem Fachmann bekannten Methoden verbessert werden.

Übliche Alkoholgemische, welche sich für die Herstellung der erfindungsgemäßen Alkylbenzoat Gemische eignen, weisen beispielsweise folgende C-Kettenverteilung auf: Summe der C6 bis C10 Fettalkohole ist kleiner gleich 3%, die Summe der C 12 Fettalkhole beträgt 60 bis 80 %, C14 Fettalkohole 15 bis 40 %, C 16 kleiner gleich 4%, Summe der Fettalkohole mit einer C-Kette größer 16 kleiner gleich 0,5 %.

Fettalkoholgemische, welche sich zur Herstellung der erfindungsgemäßen Alkylbenzoate eignen sind beispielsweise unter dem Handelsnamen Lorol® Spezial (Synative AL S) der Fa. Cognis erhältlich.

Geeignete Fettalkoholgemische weisen üblicherweise folgende Fettalkoholverteilung auf: Fettalkohole mit der C-Kette von 12 > 65 %, insbesondere > 70 %. Fettalkohole mit einer C Kette von 14 > 20 %, insbesondere größer 22 %. Der Gehalt von Fettalkoholen mit einer C-Kette von größer 16 liegt in der Regel unter 8 %, insbesondere unter 4 %.

Besonders geeignet sind Fettalkoholgemische der folgenden Zusammensetzung:
- C 12 70 - 75 %; C 14 24 - 30 %, C 16 unter 4 % oder
- C 12 65 - 71 %, C 14 22 - 28 %, C 16 unter 8 %

Umfasst sind sowohl aliphatische, aromatische, gesättigte, einfach sowie mehrfach ungesättigte, lineare und verzweigte Alkylreste. In einer bevorzugten Ausführungsform der Erfindung werden hauptsächlich (d.h. mehr als 50%, bevorzugt mehr als 60%, insbesondere mehr als 70%, besonders bevorzugt über 90% des jeweiligen Alkylrestes) aliphatische, linearen Alkohole der angegebenen C-Zahl eingesetzt, wie beispielsweise 1-Dodecanol (Laurylalkohol) als C12 Fettalkohol und Tetradecanol (Myristylalkohol) als C14 Fettalkohol.

### Kosmetische und/oder pharmazeutische Zubereitungen

Die erfindungsgemäßen Alkylbenzoat Gemische erlauben die Herstellung stabiler kosmetischer und pharmazeutischer Emulsionen.

Ein weiterer Gegenstand der vorliegenden Erfindung betrifft daher die Verwendung der erfindungsgemäßen Alkylbenzoat Gemische in kosmetischen und/oder pharmazeutischen Zubereitungen, insbesondere als Ölkörper. Die erfindungsgemäßen Alkylbenzoat Gemische können dabei, je nach Zubereitung, sowohl als alleinige Ölkörper als auch in Kombination mit weiteren Ölkörpern eingesetzt werden.

Die erfindungsgemäßen Alkylbenzoat Gemische eignen sich insbesondere zur Lösung und/oder Stabilisierung von UV-Lichtschutzfilter. Ein Gegenstand der Erfindung betrifft daher kosmetische und/oder pharmazeutische Zubereitungen, enthaltend mindestens ein Alkylbenzoat Gemisch gemäß mindestens einem der Ansprüche 1 bis 9 sowie mindestens einen UV-Lichtschutzfilter.

Erfindungsgemäß sind als **UV-Lichtschutzfilter** bei Raumtemperatur flüssige oder kristalline organische Substanzen (Lichtschutzfilter) geeignet, die in der Lage sind, ultraviolette Strahlen zu absorbieren und die aufgenommene Energie in Form längerwelliger Strahlung, z.B. Wärme wieder abzugeben. UV-Filter können öllöslich oder wasserlöslich sein. Als typische öllösliche UV-B-Filter bzw. Breitspektrum-UV A/B-Filter sind z.B. zu nennen:
➢ 3-Benzylidencampher bzw. 3-Benzylidennorcampher (Mexoryl SDS 20) und dessen Derivate, z.B. 3-(4-Methylbenzyliden)campher wie in der EP 0693471 B1 beschrieben;
➢ 3-(4'-Trimethylammonium) benzyliden- bornan-2-on-methylsulfat (Mexoryl SO)
➢ 3,3'-(1,4-Phenylendimethin)-bis (7,7- dimethyl-2-oxobicyclo-[2.2.1] heptan-1-methansulfonsäure) and salts (Mexoryl SX)
➢ 3-(4'-Sulfo)-benzyliden-bornan-2-on and salts (Mexoryl SL)
➢ Polymer von N-{(2und 4)- [2-oxoborn-3-yliden)methyl}benzyl]acrylamid (Mexoryl SW)
➢ 2-(2H-Benzotriazol-2-yl)-4-methyl-6-(2-methyl-3-(1,3,3,3-tetramethyl-1-(trimethylsilyloxy) disiloxanyl)propyl) phenol (Mexoryl XL)
➢ 4-Aminobenzoesäurederivate, vorzugsweise 4-(Dimethylamino)benzoesäure-2-ethyl-hexylester, 4-(Dimethylamino)benzoesäure-2-octylester und 4-(Dimethylamino)benzoe-säureamylester;
➢ Ester der Zimtsäure, vorzugsweise 4-Methoxyzimtsäure-2-ethylhexylester, 4-Methoxyzimtsäurepropylester, 4-Methoxyzimtsäureisoamylester, 2-Cyano-3,3-phenylzimtsäure-2-ethylhexylester (Octocrylene);
➢ Ester der Salicylsäure, vorzugsweise Salicylsäure-2-ethylhexylester, Salicylsäure-4-iso-propylbenzylester, Salicylsäurehomomenthylester;
➢ Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
➢ Ester der Benzalmalonsäure, vorzugsweise 4-Methoxybenzmalonsäuredi-2-ethylhexyl-ester;
➢ Triazinderivate, wie z.B. 2,4,6-Trianilino-(p-carbo-2'-ethyl-1'-hexyloxy)-1,3,5-triazin und 2,4,6-Tris[p-(2-ethylhexyl-oxycar-bonyl) anilino]-1,3,5-triazin (Uvinul T 150) wie in der EP 0818450 A1 beschrieben oder 4,4'-[(6-[4-((1,1-Dimethylethyl)amino-carbonyl) phenyl-amino]-1,3,5-triazin-2,4-diyl)diimino] bis(benzoesäure-2- ethylhexylester) (Uvasorb® HEB);
➢ 2,2(-Methylen-bis(6-(2H-benzotriazol-2-yl)-4- (1,1,3,3-tetramethyl-butyl)phenol) (Tinosorb M);
➢ 2,4-Bis[4-(2-ethylhexyloxy)-2-hydroxyphenyl]-6-(4- methoxyphenyl)-1,3,5- triazin (Tinosorb S);
➢ Propan-1,3-dione, wie z.B. 1-(4-tert.Butylphenyl)-3-(4'methoxyphenyl)propan-1,3-dion;
➢ Ketotricyclo(5.2.1.0)decan-Derivate, wie in der EP 0694521 B1 beschrieben;
➢ Dimethicodiethylbenzalmalonate (Parsol SLX).

Als wasserlösliche UV - Filter kommen in Frage:
➢ 2-Phenylbenzimidazol-5-sulfonsäure und deren Alkali-, Erdalkali-, Ammonium-, Alkylammonium-, Alkanolammonium- und Glucammoniumsalze;
➢ 2,2(-(1,4-Phenylen)bis(1H-benzimidazol- 4,6-disulfon-säure, Mononatriumsalz) (Neo Heliopan AP)
➢ Sulfonsäurederivate von Benzophenonen, vorzugsweise 2-Hydroxy-4-methoxybenzo-phenon-5-sulfonsäure und ihre Salze;
➢ Sulfonsäurederivate des 3-Benzylidencamphers, wie z.B. 4-(2-Oxo-3-bomylidenmethyl)-benzolsulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoyl-methan (Parsol® 1789), 1-Phenyl-3-(4'-isopropylphenyl)-propan-1,3-dion sowie Enaminverbindungen, wie beschrieben in der DE 19712033 A1 (BASF) sowie Benzoic Acid, 2-[4-(Diethylamino)-2-Hydroxybenzoyl]-, Hexyl Ester (Uvinul® A plus).

Die UV-A und UV-B-Filter können selbstverständlich auch in Mischungen eingesetzt werden. Besonders günstige Kombinationen bestehen aus den Derivaten des Benzoylmethans" z.B. 4-tert.-Butyl-4'-methoxydibenzoylmethan (Parsol® 1789) und 2-Cyano-3,3-phenylzimtsäure-2-ethyl-hexylester (Octocrylene) in Kombination mit Estern der Zimtsäure, vorzugsweise 4-Methoxyzimtsäure-2-ethylhexylester und/oder 4-Methoxyzimtsäurepropylester und/oder 4-Methoxyzimtsäureisoamylester. Vorteilhaft werden deartige Kombinationen mit wasserlöslichen Filtern wie z.B. 2-Phenylbenzimidazol-5-sulfonsäure und deren Alkali-, Erdalkali-, Ammonium-, Alkylammonium-, Alkanolammonium- und Glucammoniumsalze kombiniert.
Erfindungsgemäß bevorzugt sind UV-Lichtschutzfilter ausgewählt aus dem Anhang VII der europäischen Kosmetik-Gesetzgebung (24th Adapting Comission Directive, 29.Februar 2000)

Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und auch für die dekorative Kosmetik verwendet. Die Partikel sollten einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pig-mente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T, Eusolex® T-2000, Eusolex® T-Aqua, Eusolex® AVO, Eusolex® T-ECO, Eusolex® T-OLEO und Eusolex® T-S (Merck). Typische Beispiele für sind Zinkoxide, wie z.B. Zinc Oxide neutral, Zinc Oxide NDM (Symrise) oder Z-Cote® (BASF) oder SUNZnO-AS und SUNZnO-NAS (Sunjun Chemical Co. Ltd.). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt. Vorzugsweise wird mikronisiertes Zinkoxid verwendet. Weitere geeignete UV-Lichtschutzfilter sind der Übersicht von P.Finkel in SÖFW-Journal 122, 543 (1996) sowie Parf.Kosm. 3, 11 (1999) zu entnehmen.

Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Camosin und deren Derivate (z.B. Anserin), Carotinoide, Carotine (z.B. -Carotin, -Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, -Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis mol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. gamma-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydro-guajaretsäure, Trihydroxybutyrophenon, Harnsäure und deren Derivate, Mannose und deren Derivate, Superoxid-Dismutase, Zink und dessen Derivate (z.B. ZnO, ZnSO4) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Eine bevorzugte Ausführungsform der Erfindung betrifft kosmetischen und/oder pharmazeutische Zubereitungen, enthaltend mindestens ein Alkylbenzoat Gemisch gemäß mindestens einem der Ansprüche 1 bis 9 sowie mindestens einen UV-Lichtschutzfilter ausgewählt aus der Gruppe bestehend aus 4-Methybenzylidene Camphor, Benzophenone-3, Butyl Methoxydibenzoylmethane, Bis-Ethylhexyloxyphenol Methoxyphenyl Triazine, Methylene Bis-Benzotriazolyl Tetramethylbutylphenol, Diethylhexyl Butamido Triazone, Ethylhexyl Triazone und Diethylamino Hydroxybenzoyl Hexyl Benzoate.

Diese UV-Lichtschutzfilter sind beispielsweise unter den folgenden Handelsnamen kommerziell erhältlich: Neo Heliopan MBC (INCI: 4-Methylbenzylidene Camphor; Hersteller: Symrise); Neo Heliopan BB (INCI: Benzophenone-3, Hersteller: Symrise); Parsol 1789 (INCI: Butyl Methoxydibenzoylmethane, Hersteller: Hoffmann-La Roche (Givaudan)); Tinosorb S (INCI: Bis-Ethylhexyloxyphenol Methoxyphenyl Triazine; Hersteller: Ciba Specialty Chemicals Corporation); Tinosorb M (INCI: Methylene Bis-Benzotriazolyl Tetramethylbutylphenol, Herstelller: Ciba Specialty Chemicals Corporation), Uvasorb HEB (INCI: Diethylhexyl Butamido Triazone, Hersteller: 3V Inc.), Uvinul T 150 (INCI: Ethylhexyl Triazone, Hersteller: BASF AG), Uvinul A plus (INCI: Diethylamino Hydroxybenzoyl Hexyl Benzoate, Hersteller: BASF AG).

Die erfindungsgemäßen Alkylbenzoat Gemische können in kosmetischen und/oder pharmazeutischen Zubereitungen in Konzentrationen von 1 bis 90% eingesetzt werden. Der bevorzugte Einsatzbereich liegt zwischen 1 bis 50 %, insbesondere 2% und 20%, bezogen auf das Gesamtgewicht der kosmetischen und/oder pharmazeutischen Zubereitung. Einsatzgebiete sind beispielsweise kosmetische und/oder pharmazeutische O/W oder W/O Pflegeemulsionen, Sonnenschutzformulierung, AP/Deo Konzepte, Formulierungen für die dekorative Kosmetik, ölige Pflegezubereitungen, Tränkflüssigkeiten für Substrate, wie beispielsweise Papier- und Vliessprodukte. Exemplarisch seien genannt Wet Wipes, Taschentücher, Windeln oder Hygieneprodukte.

Die erfindungsgemäßen Alkylbenzoat Gemische eignen sich inbesondere für eine sprühbare Anwendung und/oder als Pflegeemulsion für Tissues, Papiere, Wipes, Schwämme (z.B. Polyurethanschwämme), Pflaster im Bereich Baby-Hygiene, Baby-Pflege, Hautpflege, Sonnenschutz, After-SunTreatment, Insektrepellent, Reinigung, Gesichtsreinigung und AP/Deo - Anwendung geeignet sind. Durch den Einsatz der erfindungsgemäßen Alkylbenzoat Gemische wird das sensorische Verhalten bei Applikation positiv beeinflusst.

Die kosmetischen und/oder pharmazeutischen Zubereitungen können Formulierungen zur Körperpflege sein, z. B. eine Körpermilch, Cremes, Lotionen, sprühbare Emulsionen, Produkte zur Eliminierung des Körpergeruchs etc. Die Alkylbenzoat Gemische lassen sich auch in tensidhaltigen Formulierungen wie z. B. Schaum- und Duschbädern, Haarshampoos und Pflegespülungen einsetzen. Je nach Applikationszweck enthalten die kosmetischen Formulierungen eine Reihe weiterer Hilfs- und Zusatzstoffe, wie beispielsweise Tenside, weitere Ölkörper, Emulgatoren, Perlglanzwachse, Konsistenzgeber, Verdickungsmittel, Überfettungsmittel, Stabilisatoren, Polymere, Fette, Wachse, Lecithine, Phospholipide, biogene Wirkstoffe, Deodorantien, Antitranspirantien, Antischuppenmittel, Filmbildner, Quellmittel, Insektenrepellentien, Selbstbräuner, Tyrosinaseinhibitoren (Depigmentierungsmittel), Hydrotrope, Solubilisatoren, Konservierungsmittel, Parfümöle, Farbstoffe etc., die nachstehend exemplarisch aufgelistet sind.

Als oberflächenaktive Stoffe können anionische, nichtionische, kationische und/oder amphotere bzw. zwitterionische Tenside enthalten sein. In tensidhaltigen kosmetischen Zubereitungen, wie beispielsweise Duschgelen, Schaumbädern, Shampoos etc. ist vorzugsweise wenigstens ein anionisches Tensid enthalten. Der Anteil der Tenside liegt hier üblicherweise bei etwa 1 bis 30, vorzugsweise 5 bis 25 und insbesondere 10 bis 20 Gew.-%.

Typische Beispiele für anionische Tenside sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Fettsäureethersulfate, Hydroxymisch-ethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Typische Beispiele für **nichtionische Tenside** sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, gegebenenfalls partiell oxidierte Alk(en)yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen. Typische Beispiele für kationische Tenside sind quartäre Ammoniumverbindungen, wie beispielsweise das Dimethyldistearylammoniumchlorid, und Esterquats, insbesondere quaternierte Fettsäuretrialkanolaminestersalze. Typische Beispiele für **amphotere bzw. zwitterionische Tenside** sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Hinsichtlich Struktur und Herstellung dieser Stoffe sei auf einschlägige Übersichtsarbeiten auf diesem Gebiet verwiesen. Typische Beispiele für besonders geeignete milde, d.h. besonders hautverträgliche Tenside sind Fettalkoholpolyglycolethersulfate, Monoglyceridsulfate, Mono-und/oder Dialkylsulfosuccinate, Fettsäureisethionate, Fettsäuresarcosinate, Fettsäuretauride, Fettsäureglutamate, α-Olefinsulfonate, Ethercarbonsäuren, Alkyloligoglucoside, Fettsäureglucamide, Alkylamidobetaine, Amphoacetale und/oder Proteinfettsäurekondensate, letztere vorzugsweise auf Basis von Weizenproteinen.

Körperpflegemittel, wie Cremes, Lotionen und Milchen, enthalten üblicherweise eine Reihe **weiterer Ölkörper** und Emollients, die dazu beitragen, die sensorischen Eigenschaften weiter zu optimieren. Die Ölkörper können, ja nach Art der Formulierung in einer Gesamtmenge von 1 bis 90 Gew.-%, insbesondere in einer Gesamtmenge von 1 - 50 Gew.-%, vorzugsweise 5 - 25 Gew.-% und insbesondere 5 - 15 Gew.-% enthalten sein. Als weitere Ölkörper kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen oder verzweigten C₆-C₂₂-Fettalkoholen bzw. Ester von verzweigten C₆-C₁₃-Carbonsäuren mit linearen oder verzweigten C₆-C₂₂-Fettalkoholen, wie z. B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, lsostearylstearat, lsostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von C₁₈-C₃₈-Alkylhydroxycarbonsäuren mit linearen oder verzweigten C₆-C₂₂-Feftalkoholen, insbesondere Dioctyl Malate, Ester von linearen und/oder verzweigten Fettsäuren mit mehrwertigen Alkoholen (wie z. B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, wie z. B. Dica-prylyl Carbonate (Cetiol® CC), Guerbetcarbonate auf Basis von Fettalkoholen mit 6 bis 18, vorzugsweise 8 bis 10 C Atomen, Ester der Benzoesäure mit linearen und/oder verzweigten C₆-C₂₂-Alkoholen (z. B. Finsolv® TN), welche sich von den erfindungsgemäßen Alkylbenzoate unterscheiden, lineare oder verzweigte, symmetrische oder unsymmetrische Dialkylether mit 6 bis 22 Kohlenstoffatomen pro Alkylgruppe, wie z. B. Dicaprylyl Ether (Cetiol® OE), Ringöffnungsprodukte von epoxidierten Fettsäureestern mit Polyolen.

**Fette** und **Wachse** werden den Körperpflegeprodukten als Pflegestoffe zugesetzt und auch, um die Konsistenz der Kosmetika zu erhöhen. Typische Beispiele für Fette sind Glyceride, d. h. feste pflanzliche oder tierische Produkte, die im Wesentlichen aus gemischten Glycerinestern höherer Fettsäuren bestehen. Auch Fettsäurepartialglyceride, d. h. technische Mono- und/oder Diester des Glycerins mit Fettsäuren mit 12 bis 18 Kohlenstoffatomen wie etwa Glycerinmono/dilaurat, -palmitat oder -stearat kommen hierfür in Frage. Als Wachse kommen u. a. natürliche Wachse, wie z. B. Candelillawachs, Carnaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z. B. Montanesterwachse, Sasolwachse, hydrierte Jojobawachse sowie synthetische Wachse, wie z. B. Polyalkylenwachse und Polyethylenglycolwachse in Frage. Neben den Fetten kommen als Zusatzstoffe auch fettähnliche Substanzen, wie Lecithine und Phospholipide in Frage. Als Beispiele für natürliche Lecithine seien die Kephaline genannt, die auch als Phosphatidsäuren bezeichnet werden und Derivate der 1,2-Diacyl-sn-glycerin-3-phosphorsäuren darstellen. Dem gegenüber versteht man unter Phospholipiden gewöhnlich Mono- und vorzugsweise Diester der Phosphorsäure mit Glycerin (Glycerinphosphate), die allgemein zu den Fetten gerechnet werden. Daneben kommen auch Sphingosine bzw. Sphingolipide in Frage.

Als **Verdickungsmittel** eignen sich beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethyl- und Hydroxypropylcellulose, Polyvinylalkohol, Polyvinylpyrrolidon und Bentonite wie z. B. Bentone^{®} Gel VS-5PC (Rheox).

Unter **biogenen Wirkstoffen** sind beispielsweise Tocopherol, Tocopherolacetat, Tocopherolpalmitat, Ascorbinsäure, (Desoxy)Ribonucleinsäure und deren Fragmentierungsprodukte, β-Glucane, Retinol, Bisabolol, Allantoin, Phytantriol, Panthenol, AHA-Säuren, Aminosäuren, Ceramide, Pseudoceramide, essentielle Öle, Pflanzenextrakte, wie z. B. Prunusextrakt, Bambaranussextrakt und Vitaminkomplexe zu verstehen.

**Desodorierende Wirkstoffe** wirken Körpergerüchen entgegen, überdecken oder beseitigen sie. Körpergerüche entstehen durch die Einwirkung von Hautbakterien auf apokrinen Schweiß, wobei unangenehm riechende Abbauprodukte gebildet werden. Dementsprechend eignen sich als deosodorierende Wirkstoffe u. a. keimhemmende Mittel, Enzyminhibitoren, Geruchsabsorber oder Geruchsüberdecker.

Als **Insekten-Repellentien** kommen beispielsweise N,N-Diethyl-m-toluamid, 1,2-Pentandiol oder 3-(N-n-Butyl-N-acetyl-amino)-propionsäureethylester), welches unter der Bezeichnung Insect Repellent^{®} 3535 von der Merck KGaA vertrieben wird, sowie Butylacetylaminopropionate in Frage.

Als **Selbstbräuner** eignet sich Dihydroxyaceton. Als Tyrosinhinbitoren, die die Bildung von Melanin verhindern und Anwendung in Depigmentierungsmitteln finden, kommen beispielsweise Arbutin, Ferulasäure, Kojisäure, Cumarinsäure und Ascorbinsäure (Vitamin C) in Frage.

Als **Konservierungsmittel** eignen sich beispielsweise Phenoxyethanol, Formaldehydlösung, Parabene, Pentandiol oder Sorbinsäure sowie die unter der Bezeichnung Surfacine® bekannten Silberkomplexe und die in Anlage 6, Teil A und B der Kosmetikverordnung aufgeführten weiteren Stoffklassen.

Als **Parfümöle** seien genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen, Harzen und Balsamen. Weiterhin kommen tierische Rohstoffe, wie beispielsweise Zibet und Castoreum sowie synthetische Riechstoffverbindungen vom Typ der Ester, Ether, Aldehyde, Ketone, Alkohole und Kohlenwasserstoffe in Frage.

Als **Perlglanzwachse,** insbesondere für den Einsatz in tensidischen Formulierungen, kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxy-substituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

Als **Überfettungsmittel** können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

Als **Stabilisatoren** können Metallsalze von Fettsäuren, wie z. B. Magnesium-, Aluminium- und/oder Zinkstearat bzw. -ricinoleat eingesetzt werden.

Zur Verbesserung des Fließverhaltens können ferner **Hydrotrope,** wie beispielsweise Ethanol, lsopropylalkohol, oder Polyole eingesetzt werden. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein.

### Beispiele

### Erfindungsgemäßes Beispiel A- Herstellung

Das Fettalkohol Gemisch Lorol®Spezial (Fa. Cognis; Fettalkoholverteilung C 12 70-75 %, C 14 24-30%, C16 unter 4%) und Benzoesäure wurden unter Sn-(II)-Katalyse bei 180 bis 230 °C unter Abspaltung von Wasser bei Normaldruck und später bei leichtem Vakuum verestert. Anschliessend wurde der überschüssige Alkohol unter Vakuum abdestiliiert und das Reaktionsgemisch abgekühlt. Der Katalysator wurde entfernt und das Produkt desodoriert.

### Vergleichsbeispiel B

Als Vergleichsbeispiel dient das kommerziell erhältiche Produkt Finsolv®TN (Fa. Finetex). Die folgende Tabelle zeigt die C-Kettenverteilung des erfindungsgemäßen Beispiels A sowie des Vergleichsbeispiels B.

| C-Kettenverteilung | Beispiel A (erfindungsgemäß) | Beispiel B (Vergleich) |
|---|---|---|
| C 12, linear | 70-75 % | 22,7 % |
| C12, verzweigt | < 1% | 3,1 % |
| Summe C12 | 70-75 % | 25,8 % |
| C 13, linear | | 31,8 % |
| C 13 , verzweigt | | 4,5 % |
| Summe C 13 | < 1 % | 36,3 % |
| C 14, linear | 24-30 % | 17,5 % |
| C 14, verzweigt | < 1% | 3,7 % |
| Summe C 14 | | 21,2 % |
| C 15, linear | | 14,5 % |
| C 15 verzweigt | | 2,1 % |
| Summe C 15 | < 1 % | 16,6 % |
| ≥C16 | < 4 % | 0,1 % |

### Beispiel 1 Sensorische Bewertung

Die sensorische Bewertung des erfindungsgemäßen Beispiels A erfolgte zum Vergleichsbeispiel B: Ein Panel bestehend aus 12 Experten führte die sensorische Bewertung durch. Abgefragt wurden die folgenden Kriterien bezogen auf das Endgefühl auf der Haut: Weichheit, Glätte, Pflege. Gleichzeitig wurde auch die Akzeptanz abgefragt.

Die Bewertung dieser Kriterien erfolgte mit Noten von 1 (wenig) bis 7 (viel)

| Kriterien | Erfindungsgemäßes Beispiel A | Vergleichsbeispiel B Finsolv®TN (Fa. Finetex) |
|---|---|---|
| Glätte | 7 | 5 |
| Weichheit | 6 | 4 |
| Pflege | 7 | 5 |
| Akzeptanz | 7 | 4 |

Das erfindungsgemäße Beispiel A zeigt gegenüber dem Stand der Technik eine verbesserte Sensorik bei einer hohen Akzeptanz.

### Löslichkeit verschiedener UV-Lichtschutzfilter

Zur Bestimmung der Löslichkeit wurden verschiedene, kommerziell erhältliche UV-Lichtschutzfilter in dem erfindungsgemäßen Alkylbenzoat Gemisch (gemäß Beispiel A) bzw. in dem kommerziell erhältliche C12-C15 Alkylbenzoate, Finsolv®TN in der Wärme bei 80°C gelöst und 1 Woche bei 15°C gelagert. Die folgende Tabelle zeigt die Menge an UV Filter in Gew. %, die nach Lagerung von 1 Woche bei 15 °C klar in Lösung bleiben.

| | Löslichkeit [Gew. %] |
|---|---|
| Alkylbenzoat Gemisch nach Beispiel 1 plus Uvinul® T 150 | 10 |
| Finsolv®TN plus Uvinul T 150 | 5 |
| Alkylbenzoat Gemisch nach Beispiel 1 plus Neoheliopan® BB | 15 |
| Finsolv®TN1 plus Neoheliopan BB | 10 |
| Alkylbenzoat Gemisch nach Beispiel 1 plus Parsol® 1789 | 15 |
| Finsolv®TN plus Parsol 1789 | 10 |

| | |
|---|---|
| Uvinul®T 150; INCI: Ethylhexyl Triazone (BASF AG) Neo Heliopan®BB; INCI:. Benzophenone-3 (Symrise) Parsol®1789; INCI: Butyl Methoxydibenzoylmethane (DSM Nutritional Products) | |

### Kosmetische Zubereitungen: Formulierungen für Spray - und Wipe - Anwendungen sowie für AP / Deo Konzepte

Die Rezepturen 1 bis 26 beschreiben stabile Formulierungen auf Basis der erfindungsgemäßen Ölkomponente, insbesondere des Herstellungsbeispiels A, die besonders für eine Anwendung als Pump-oder Aerosolspray und/oder als Pflegeemulsion für Tissues, Papiere, Wipes, Schwämme (z.B. Polyurethanschwämme), Pflaster im Bereich Baby-Hygiene, Baby-Pflege, Hautpflege, Sonnenschutz, After-SunTreatment, Insektrepellent, Reinigung, Gesichtsreinigung und AP/Deo - Anwendung geeignet sind. Bei Anwendung als Aerosolspray sind Treibgase wie Propan, Butan, iso.Butan oder Mischungen besonders geeignet. Durch den Einsatz der erfindungsgemäßen Ölkomponente wird das sensorische Verhalten bei Applikation positiv beeinflusst. Die Mengenangaben beziehen sich jeweils auf Gew.-% der handelsüblichen Substanzen in der Gesamtzusammensetzung.

**Tabelle 1: Rezepturen 1 bis 13**

| **Komponenten INCI (Handels-name)** | **1** | **2** | **3** | **4** | **5** | **6** | **7** | **8** | **9** | **10** | **11** | **12** | **13** |
|---|---|---|---|---|---|---|---|---|---|---|---|---|---|
| Emulgade^{®} SE | | | | | | | | | | | | 10.7 | 5.1 |
| Eumulgin^{®} B2 | | | | | | | | | | | | 5.8 | 3.4 |
| Emulgade^{®} PL 68/50 | 1 | | 1 | 1 | | 2 | 2 | | 2 | | 2 | | |
| Eumulgin^{®} VL 75 | | 1 | | | 1 | | | 3 | | 2.5 | | | |
| Lanette^{®} E | 1 | 1 | 1 | | 1 | | | | 1 | 1 | | | |
| Emulgade^{®} SUCRO | 1 | 1.5 | | | | | 1 | | 9 | | | 0.5 | 0.5 |
| **Erfindungsgemäße Ölkomponente** | **5** | **4** | **8** | **3** | **5** | **8** | **4** | **2** | **4** | **3** | **5** | **10** | **2** |
| Cetiol^{®} CC | 5 | 5 | 5 | | | | | 4 | | 5 | 3 | 4 | |
| Myritol^{®} 331 | 3 | 4 | | 4 | 4 | | | | 5 | | | 3 | 3 |
| Cetiol^{®} OE | | | | | 5 | | 3 | | 2 | | | | |
| Cetiol^{®} B | | | | 4 | | | | 4 | | 4 | | | |
| Cosmedia^{®} DC | 1 | 1 | 1 | 1 | 1 | 1.5 | 1.5 | 2 | 3 | 2 | 1.5 | 2 | 2 |
| Insect Repellent^{®} 3535 | | | | | | | 2 | | | | 5 | 5 | |
| Copherol^{®} F1300 C | 1 | 1 | 1 | 1 | 1 | 1 | 1 | 1 | 1 | 1 | 1 | 1 | 1 |
| Zink oxide NDM | 5 | 5 | 5 | 5 | 5 | | 2 | | 5 | 3 | | | |
| Eusolex^{®} T 2000 | | | | 5 | 5 | | 2 | 3 | 5 | 2 | | | |
| Neo Heliopan^{®} AV | 7.5 | 7.5 | 7.5 | | | | 3 | 1 | 3 | 5 | | 5 | 5 |
| Parsol^{®} 1789 | | | | | | 2 | 2 | | | 1 | 2 | 2 | |
| Neo Heliopan^{®} MBC | | | | | | | 2 | | | | | | 2 |
| Uvinul^{®} T 150 | | | | | | 1 | 1 | 2 | | | 1 | | |
| Uvasorb^{®} HEB | | | | | | 1 | 1 | 2 | | | 1 | 2 | |
| Neo Heliopan^{®} Hydro - Na-Salz, 15% wäßrige Lsg. | | | | | | | | | | | | | 13.3 |
| Glycerin | 3 | 3 | 3 | 3 | 3 | 3 | 3 | 3 | 3 | 3 | 3 | 5 | 5 |
| Veegum^{®} plus | 0.75 | 0.75 | | 0.5 | 0.5 | | 0.5 | | | 0.35 | | | |
| Keltrol T | 0.25 | 0.25 | | 0.5 | 0.5 | | 0.5 | | | 0.35 | | | |
| Cosmedia^{®} SP | | | 0.1 | | | 0.1 | 0.2 | | | | 0.1 | | |
| Permulen^{®} TR-2 Polymer | | | | | | | | 0.2 | 0.1 | | | | |
| Wasser, Parfum, Konservierungsmittel | q.s. | q.s. | q.s. | q.s. | q.s. | q.s. | q.s. | q.s. | q.s. | q.s. | q.s. | q.s. | q.s. |

**Tabelle 2 Rezepturen 14 bis 26**

| **Komponenten INCI (Handels-name)** | **14** | **15** | **16** | **17** | **18** | **19** | **20** | **21** | **22** | **23** | **24** | **25** | **26** |
|---|---|---|---|---|---|---|---|---|---|---|---|---|---|
| Emulgade^{®} SE | 3.7 | 3.7 | | | | | | | | | | 4.9 | 4.1 |
| Eumulgin^{®}B1 | 1.3 | 1.3 | | | | | | | | | | | |
| Eumulgin^{®}B2 | | | | | | | | | | | | 1.1 | 0.9 |
| Emulgade^{®} PL 68/50 | | | 5 | 1 | 1 | 1 | 1 | 3 | | | | | |
| Eumulgin^{®} VL 75 | | | | | | | | | 3 | 5 | 5 | | |
| Lanette^{®} E | | | | 0.25 | 0.25 | 0.25 | 0.25 | .25 | | | | | |
| Amphisol K | | | 0.5 | | | | | | | | | | |
| Emulgade^{®}SUCRO | | | 0.5 | | | | | | 0.5 | | | 0.5 | 1 |
| **erfindungsgemäße Ölkomponente** | 4 | 5 | 6 | 8 | 5 | 8 | 8 | 10 | 7 | 4 | 10 | 5 | 5 |
| Cetiol^{®} CC | 5 | | 5 | | | | | | 2.5 | 4 | 4 | 5 | 5 |
| Cetiol^{®} LC | | | | 1 | 1 | 1 | 1 | 1 | | | | | |
| Myritol^{®} 312 | | | | 1 | 1 | 1 | 1 | 1 | | | | | |
| Myritol^{®} 331 | | | | | | | | | | 4 | 4 | | |
| Cetiol^{®} SN | 3 | 3 | 3.5 | | | | | | | | | | |
| Eutanol^{®} G | | | | | | | | | 3.5 | 2 | 2 | | |
| Eutanol^{®} G16 | | | | 1 | 1 | 1 | 1 | 1 | | | | | |
| Cegesoft^{®} PS6 | | 1.5 | 1.5 | | | | | | | | | | |
| Cegesoft^{®} PFO | 1.5 | | | | | | | | | | | | |
| Silikonöl Wacker AK^{®} 350 | | | | 0.5 | 0.5 | 0.5 | 0.5 | 0.5 | | | | | |
| Cosmedia^{®} DC | 1 | | 1.5 | | | | | 1.5 | | 2,5 | 2,5 | | 0.5 |
| Hydagen^{®} C.A.T | | | | | | | | | | | | 1.5 | |
| Copherol^{®} F 1300 C | | | | | | | | | 0.5 | 0.5 | 0.5 | | |
| Copherol^{®} 1250 C | 0.5 | 0.5 | | | | | | | | | | | |
| Ethanol | | | | | | | | | | | 5 | | |
| Locron^{®} L | | | | | | | | | | | | | 40 |
| Hydagen^{®} DCMF | | | | | | | | | | | | 0.1 | |
| Glycolic Acid | | | | | | | | | | | | 0.04 | |
| Glycerin | 2 | 2 | 2 | 3 | 3 | 3 | 3 | 3 | 2 | 2 | 2 | 3 | 2 |
| Potassium Hydroxyde, 20% wäßrige Lsg. | | | | | | 0.3 | 0.2 | 0.1 | 0.4 | 0.3 | 0.5 | | |
| Hispagel^{®} 50 | | | | | | | | | | 10 | | | |
| Carbomer | | | | | | | 0.1 | | 0.2 | | 0.2 | | |
| Cosmedia^{®} SP | | | | | 0.15 | | | | | | | | |
| Permulen^{®} TR-2 Polymer | | | | | | 0.15 | | 0.05 | | | | | |
| Wasser, Parfum, Konservierungsmittel | q.s. | q.s. | q.s. | q.s. | q.s. | q.s. | q.s. | q.s. | q.s. | q.s. | q.s. | q.s. | |

**Tabelle 3: Rezepturen 27 bis 33 (Formulierungen für AP/Deo)**

| **Komponenten INCI (Handelsname)** | **27** | **28** | **29** | **30** | **31** | **32** | **33** |
|---|---|---|---|---|---|---|---|
| Emulgade^{®} SE-PF | 6 | | 4,5 | | | 6 | |
| Ceteareth-12 (Eumulgin^{®}B1) | | | | | | | |
| Ceteareth-20 (Eumulgin^{®}B2) | | | 1 | | | | |
| Emulgade^{®} CM | | | | | 20 | | |
| Lameform^{®} TGI | | 3 | | | | | |
| Novata^{®} AB | | | | | | | 4 |
| Lanette^{®} 18 | | | | 14,7 | | | |
| Cutina^{®} HR | | | | 3,7 | | | 6,5 |
| Dehymuls^{®} PGPH | | 1 | | | | | |
| Lanette^{®} E | 0,3 | | | | | 0,3 | |
| Lanette^{®} 22 | 2 | | | | | 4 | |
| Emulgade^{®} SUCRO | 0.8 | 1.3 | | 1 | | | 2 |
| **Erfindungsgemäße Ölkomponente** | 4 | 4 | 5 | 5 | 4 | 4 | 15 |
| Cetiol^{®} CC | | 3 | | | | | |
| Cetiol^{®} OE | 2 | | | 4 | | 3 | 9 |
| Myritol^{®} 331 | | | | | | | |
| Cetiol^{®} S | | | 5 | 14,7 | | | 20 |
| Dow Corning^{®} 246 Fluid | 3 | 5 | | 34 | | 2 | 14 |
| SFE^{®} 839 (GE Bayer) | | 3 | | | | | |
| Silikonöl Wacker AK^{®} 350 | 1 | | | | | | |
| Cosmedia^{®} DC | 1 | 1 | 1 | 1 | 1 | 1 | 1 |
| Hydagen^{®} C.A.T | | | 2 | | | | |
| Eumulgin^{®} HRE 40 | | | | | 1 | | |
| Copherol^{®} 1250 C | | | | 1 | | | |
| Rezal^{®} 36 | 30 | 40 | | 22,9 | | 30 | 25 |
| Locron^{®} L | | | 10 | | | | |
| Hydagen^{®} DCMF | 0,05 | | | | | | |
| Glycolic Acid | 0,02 | | | | | | |
| Glycerin | | 5 | 5 | | | | |
| Propylene Carbonate | | | | | | | 0,5 |
| Bentone^{®} 18 | | | | | | | 1 |
| Talkum | | | | | | 5 | 5 |
| MgSO4x 7H2O | | 1 | | | | | |
| Wasser Phase II | 46,7 | | 35 | | | | |
| Wasser, Parfum, Konservierungsmittel | q.s. | q.s. | q.s. | q.s. | q.s. | q.s. | q.s. |

| | | | | | | | |
|---|---|---|---|---|---|---|---|
| 27 - Antiperspirant / Deo Creme 28 - Antiperspirant Creme (W/O) 29 - Antiperspirant / Deo Spray 30 - Antiperspirant Stift mit Vitamin E 31 - Deodorant Wipe - Formulierung 32 - Antiperspirant Creme 33 - Antiperspirant Creme «Soft Solid » | | | | | | | |

In der Tabelle 4 werden Sonnenschutzformulierungen vom Typ O/W beschrieben, in der Tabelle 5 werden Pflegeemulsionen beschrieben. Durch den Einsatz der erfindungsgemäßen Ölkomponente, insbesondere des Herstellungsbeispiels A, wird das sensorische Verhalten bei Applikation positiv beeinflusst. Die Mengenangaben beziehen sich jeweils auf Gew.-% der handelsüblichen Substanzen in der Gesamtzusammensetzung.

**Tabelle 4: O/W-Sonnenschutzemulsionen**

| **Komponente** | **1** | **2** | **3** | **4** | **5** | **6** | **7** | **8** | **9** | **10** | **11** |
|---|---|---|---|---|---|---|---|---|---|---|---|
| **L = Lotion, C = Creme, S = Spray** | L | C | L | C | L | C | S | C | C | L | L |
| Eumulgin^{®} VL 75 | 2 | | | | 3 | | | | 1 | | |
| Eumulgin^{®} B2 | | | | 2 | | | | | | 1 | |
| Tween^{®} 60 | | | | | | | | | | 1 | |
| Cutina^{®} E 24 | | | | 0.5 | | | | | | | |
| Hostaphat^{®} KL 340 N | | | | | | | | | | | 0.5 |
| Eumulgin^{®} SG | | | 0,5 | | | 0,5 | | 0,3 | 0,1 | | |
| Lanette^{®} E | | | | | | | 0.1 | | 0.5 | | |
| Amphisol^{®} K | 0.5 | | | | | | 1 | | | | |
| Natriumstearat | | | | | 1 | | | | | | |
| Emulgade^{®} PL 68/50 | | 2 | 1 | | | 3 | | | | | |
| Emulgade^{®} SUCRO | | 2 | | | | 1 | 1 | | 3 | | 1 |
| Tego^{®} Care 450 | | 2 | | | | | | | 2 | | 1 |
| Cutina^{®} MD | | | | 2 | 1 | 3 | | | | | 1 |
| Lanette^{®} 14 | | 1 | | | | | | | | | |
| Lanette^{®} O | | | | 2 | | | | 2 | 1 | 1 | |
| Cutina^{®} PES | 1 | 1 | | 2 | | | | | | 1 | |
| Allianz^{®} OPT | 1 | | | 1 | 1 | | | 2 | | | 2 |
| Cosmedia^{®} DC | | 1.5 | 2 | | | 1.5 | 2 | | 1.5 | 1.5 | |
| Emery^{®} 1780 | | | | 1 | 1 | | | | | | |
| Lanolin, wasserfrei, USP | | | | | | 1 | 1 | | | | |
| **erfindungsgemäße Ölkomponente** | **6** | **2** | **4** | **7** | **3** | **7** | **6** | **6** | **4** | **4** | **5** |
| Myritol^{®} PC | | | | | | | | | 5 | | |
| Myritol^{®} 331 | 6 | | 4 | | | 5 | 8 | | | 10 | 8 |
| Finsolv^{®} TN | | | | | 5 | | | 3 | 3 | | |
| Cetiol^{®} CC | 6 | | 6 | | | 5 | 5 | | | | |
| Cetiol^{®} OE | | | | | 2 | | | | | | 2 |
| Dow Coming DC^{®} 244 | | 2 | | | 1 | | | | | | |
| Dow Coming DC^{®} 2502 | | 1 | | | 1 | | | 3 | | | |
| Ceraphyl^{®} 45 | | | | | | | | | | 2 | 2 |
| Silikonöl Wacker AK^{®} 350 | | | | | 1 | | | | | | |
| Cetiol^{®} 868 | | 2 | | | | | | | | | |
| Cetiol^{®} J 600 | | 2 | | | | | | | | | |
| Mineralöl | | | | 5 | | | | | | | |
| Cetiol^{®} B | 4 | | 4 | | | | | 4 | | | |

| | | | | | | | | | | | |
|---|---|---|---|---|---|---|---|---|---|---|---|
| Eutanol^{®} G | | 3 | | | | 3 | | | | | |
| Eutanol^{®} G 16 S | 3 | | | | | | | | | | |
| Cetiol^{®} PGL | | | | | | | | | | | |
| Photonyl^{®} LS | | | | | | | | | | 2 | |
| Panthenol | 1 | | | | | | | | | | |
| Bisabolol | 0,2 | | | | | | | | | | |
| Tocopherol / Tocopherylacetat | 1 | | | | | | | | | | |
| Neo Heliopan^{®} Hydro (Na-Salz) | | | | | | | | | | 3 | |
| Eusolex^{®} OCR | 6 | | 9 | | 5 | 7 | 9 | | 4 | | 7 |
| Neo Heliopan^{®} AP (Na-Salz) | | | | 0. 5 | | 1 | | | | | |
| Neo Heliopan^{®} BB | | | | | | | | 1 | 1 | | 1 |
| Neo Heliopan^{®} MBC | | 2 | | 1 | | | | 3 | 1 | | 3 |
| Neo Heliopan^{®} OS | 2 | | | | | | | | 7 | | |
| Neo Heliopan^{®} E1000 | | 4 | | | | | | 5 | | | |
| Neo Heliopan^{®} AV | | 4 | 7.5 | 5 | | | | 5 | 4 | 7.5 | |
| Uvinul^{®} A PLUS | | | | | 1 | | 2 | | | | |
| Uvinul^{®} T 150 | 1 | | | | | | | | 1.3 | 1 | 1 |
| Tinosorb^{®} M | | 2 | | | 2 | | 2 | | | | |
| Tinosorb^{®} S | | 1 | | | 2 | | 2 | | | | |
| Parsol^{®} 1789 | 1 | | | | | | | | 2 | | 1 |
| Z-Cote^{®} HP 1 | 7 | 2 | 5 | | | 7 | 5 | | 6 | 2 | |
| Eusolex^{®} T 2000 | 5 | 2 | | | 1 0 | | | 10 | | 2 | |
| Veegum^{®} Ultra | 1.5 | | 1.5 | | | 1. 5 | 1.2 | | 1 | | |
| Keltrol^{®} T | 0.5 | | 0.5 | | | 0. 5 | 0.4 | | 0.5 | | |
| Cosmedia^{®} SP | | | 0.2 | 0. 3 | | | 0.1 | | | 0.2 | |
| Pemulen^{®} TR 2 | | 0.3 | | 0. 3 | | | | 0.2 | | | 0. 3 |
| Ethanol | | 5 | | 8 | | | | | | | |
| Butylenglykol | 1 | | | 3 | 3 | | | | | 8 | 1 |
| Glycerin | 2 | 4 | 3 | 3 | | 3 | 3 | 3 | 5 | | 3 |
| Wasser/ Konservierungsmittel/ NaOH | ad 100/ q.s./ q.s | | | | | | | | | | |

**Tabelle 5: O/W-Pflegeemulsionen**

| **Komponente** | **12** | **13** | **14** | **15** | **16** | **17** | **18** | **19** | **20** | **21** | **22** |
|---|---|---|---|---|---|---|---|---|---|---|---|
| **L = Lotion, C = Creme** | C | C | L | C | L | C | L | L | L | L | C |
| Eumulgin^{®} VL 75 | | | 5 | | 4 | | | | | | 2 |
| Generol^{®} R | | | | | | 2 | | | | | |
| Eumulgin^{®} B2 | | | | | | | | | | 1 | |
| Tween^{®} 60 | | | | | | | | | | 1 | |
| Cutina^{®} E 24 | | | | 0.5 | | | | | | | |
| Eumulgin^{®} SG | | | 0,1 | 0,5 | | 0,4 | | 0,2 | 0,1 | | |
| Lanette^{®} E | 0.5 | | | | | | | | | | |
| Amphisol^{®} K | 0,5 | 0.5 | | | | | | | | | |
| Natriumstearat | | | | | 1 | | | | | | |
| Emulgade^{®} PL 68/50 | | 2 | | 2 | | | | 3 | 4 | | |
| Emulgade^{®} SUCRO | 2 | 1 | 1 | 1 | | | | | | | 2 |
| Tego^{®} Care 450 | | 1 | | | | | | | 1 | | |
| Cutina^{®} MD | 2 | 1 | 1 | 1 | | 5 | | | | 2 | |
| Lanette^{®} 14 | | | | | 1 | | | 2 | | 1 | |
| Lanette^{®} O | 2 | | | 2 | 1 | 3 | 1 | | 1 | 1 | 3 |
| Cutina^{®} PES | 1 | 2 | | 3 | 1 | | | | | | 3 |
| Novata^{®} AB | | | | | | | | | 1 | 1 | |
| Emery^{®} 1780 | | | | | | | | | | | 0.5 |
| Lanolin, wasserfrei, USP | | | | | | 4 | | | | | |
| Cosmedia^{®} DC | | | 2 | | | 1.5 | | | 1 | 1 | |
| Cetiol^{®} SB 45 | | | | | | | 2 | | | | |
| Cegesoft^{®} C 17 | 2 | | | | | | | | | | |
| **erfindungsgemäße Ölkomponente** | **5** | **5** | **4** | **4** | **3** | **4** | **5** | **4** | **5** | **10** | **2** |
| Myritol^{®} PC | 6 | | | | | 5 | | | | | |
| Myritol^{®} 331 | 2 | | 5 | | | | 2 | | | | 3 |
| Finsolv^{®} TN | | | | 3 | 5 | | | 3 | 3 | | 1 |
| Cetiol^{®} CC | | | | 3 | | | 4 | 3 | | | |
| Cetiol^{®} OE | | | | | 2 | | 2 | | 5 | | |
| Dow Coming DC^{®} 245 | | 2 | | | 1 | 4 | | | | 8 | 2 |
| Dow Coming DC^{®} 2502 | | 1 | | | 1 | | | | | | 3 |
| Prisorine^{®} 3758 | 3 | | | | | | | | | | 2 |
| Silikonöl Wacker AK^{®} 350 | | | | | 1 | | | | | | 1 |
| Cetiol^{®} 868 | | 2 | | | | | | | | | |
| Cetiol^{®} J 600 | | 2 | | 2 | | | | | | | |
| Ceraphyl^{®} 45 | | | | | | | 3 | | | | |
| Cetiol^{®} SN | | | | 5 | | | | | | | |
| Cetiol^{®} B | | | 5 | | | 5 | | 4 | | | 3 |
| Eutanol^{®} G | | 3 | 5 | | 5 | | | | | | |
| Cetiol^{®} PGL | | | | | | | | 5 | 21 | | |
| Dry Flo^{®} Plus | | 1 | | | | | | | | | 1 |
| SFE 839 | 1 | 1 | | | | | | | | | |
| Mandelöl | | | | | | 2 | | | | | |
| Photonyl^{®} LS | | | | | | 2 | | | | | |
| Panthenol | 1 | | | | | | | | | | |
| Bisabolol | 0.2 | | | | | | | | | | |
| Tocopherol / Tocopherylacetat | 1 | | | | | | | | | | |
| Veegum^{®} Ultra | | | | | | | | | 1 | | |
| Keltrol^{®} T | | | | | | | | | 0.5 | | |
| Cosmedia^{®} SP | 0.5 | | | | | 0.5 | 0.5 | 0.2 | | | 0.5 |
| Carbopol^{®} ETD 2001 | | 0.3 | | 0.3 | | | | | | | |
| Pemulen^{®} TR 2 | | | 0.3 | | | 0.3 | | | | | |
| Ethanol | | 5 | | 8 | | | | | | | 10 |
| Butylenglykol | 5 | | 2 | 3 | 3 | | | | | 8 | |
| Glycerin | 2 | 4 | 3 | 3 | | 7 | 5 | 3 | 5 | | |
| Wasser, Konservierungsmittel, NaOH | ad 100, q.s. (pH 6,5 - 7,5) | | | | | | | | | | |

### Formulierungen für den Sonnenschutz und die Hautpflege vom Typ Wasser in Öl

In Tabelle 6 werden Sonnenschutzformulierungen vom W/O Emulsionstyp, in Tabelle 7 werden Pflegeemulsionen beschrieben. Durch den Einsatz der erfindungsgemäßen Ölkomponente, insbesondere des Herstellungsbeispiels A, wird das sensorische Verhalten bei Applikation positiv beeinflusst. Die Mengenangaben beziehen sich jeweils auf Gew.-% der handelsüblichen Substanzen in der Gesamtzusammensetzung.

**Tabelle 6 : W/O - Sonnenschutzformulierungen**

| **Ingredient** | **23** | **24** | **25** | **26** | **27** | **28** | **29** | **30** | **31** | **32** | **33** |
|---|---|---|---|---|---|---|---|---|---|---|---|
| **L = Lotion; C = Creme** | C | L | C | L | C | L | L | L | L | C | C |
| Dehymuls^{®} PGPH | 4 | 2 | 1 | 3 | 3 | 1 | 1 | 2 | 2 | 4 | 1 |
| Monomuls^{®} 90-O18 | | | 2 | | | | | | | | |
| Lameform^{®} TGI | 2 | | 4 | | 3 | | | | | 1 | 3 |
| Abil^{®} EM 90 | | | | | | | 4 | | | | |
| Isolan^{®} PDI | | | | | | 4 | | 2 | | | |
| Zinkstearat | 1 | | | 1 | 1 | | | 1 | | 1 | |
| Bienenwachs | 1 | | 5 | 1 | | | | 5 | | 7 | 5 |
| Tego^{®} Care CG | | | | | 1 | | | | | | 0.5 |
| Emulgade^{®} SUCRO | 1 | 1 | | | 1 | | | 1 | | 1 | |
| Prisorine^{®} 3505 | | | 1 | | | 1 | 1 | | | | 1 |
| Cosmedia DC | 3 | 4 | 2 | 1 | 1 | 2 | 2 | 2 | 3 | 1 | 1 |
| erfindungsgemäße Ölkomponente | 5 | 4 | 4 | 3 | 2 | 4 | 3 | 4 | 2 | 3 | 5 |
| Myritol^{®} 331 | 2 | | | | 3 | 6 | | | | | 3 |
| Finsolv^{®} TN | | | | 5 | | | 2 | | | | |
| Cetiol^{®} CC | 5 | | 2 | | 4 | 2 | | | 2 | 3 | 5 |
| Tegosoft DEC | | 4 | | 3 | | | 5 | 5 | | | |
| Cetiol^{®} OE | | | | | 4 | | 5 | | 4 | 2 | |
| Dow Corning^{®} DC 244 | | | 3 | | | | 2 | | 2 | 4 | |
| Dow Corning^{®} DC 2502 | 1 | | 1 | | 2 | 1 | | | | | 1 |
| Silikonöl Wacker AK 350 | | 1 | | 4 | | | | 3 | | | |
| Cetiol^{®} PGL | | 3 | | | | 2 | | | 4 | | |
| Cophero^{®} F 1300 | 1 | | | | | | | | | | |
| Magnesium sulfat x 7 H₂O | 1 | | | | | | | | | | |
| Neo Heliopan^{®} Hydro (Na-Salz) | 2 | | 2.2 | | 3 | 3 | | | 1 | | 2 |
| Neo Heliopan^{®} 303 | | 5 | | | | | | | 4 | | 4 |
| Neo Heliopan^{®} BB | 2 | | | | | | 1 | 1 | | | |
| Uvasorb^{®} HEB | 1 | | | 1 | 1 | | | | | | 2 |
| Neo Heliopan^{®} MBC | 2 | | | | | 2 | 2 | 2 | | | |
| Uvinul^{®} A plus | | | | | 2 | | | | 3 | 3 | |
| Neo Heliopan^{®} AP (Na-Salz) | | 2 | 2 | | 1 | | | | 1 | | 6 |
| Neo Heliopan^{®} AV | 3 | | 4 | 6 | 4 | 7.5 | 4 | 5 | | | 1 |
| Uvinul^{®} T 150 | 1 | 1 | | | 2.5 | | | 1 | | | |
| Parsol^{®} 1789 | 2 | 1 | | | | | 2 | | 2 | 2 | |
| Zinkoxid NDM | | | | | | 10 | | 3 | | | 4 |
| Tinosorb^{®} M | | 3 | | 3 | | | | 2 | | 2 | |
| Tinosorb^{®} S | | 3 | | 3 | | | | 2 | | 2 | |
| Eusolex^{®} T Aqua | | | 8 | | | | | 5 | | | |
| Eusolex^{®} T 2000 | | | | | 5 | | 3 | 3 | | | 4 |
| Ethanol | | | | | | | | | | 8 | |
| Glycerin | 5 | 3 | 3 | 3 | 5 | 3 | 2 | 3 | 10 | 4 | 3 |
| Wasser, Konservierungsmittel | ad 100, q.s. | | | | | | | | | | |

**Tabelle 7: W/O-Pflegeemulsionen**

| **Komponente** | **34** | **35** | **36** | **37** | **38** | **39** | **40** | **41** | **42** | **43** | **44** |
|---|---|---|---|---|---|---|---|---|---|---|---|
| **L = Lotion. C = Creme** | C | L | C | L | C | L | L | L | C | C | C |
| Dehymuls^{®} PGPH | 1 | 3 | 1 | 2 | 3 | 1 | 1 | 2 | 1 | 1 | 1 |
| Monomuls^{®} 90-O18 | 2 | | | | | | | | 2 | | 2 |
| Lameform^{®} TGI | 4 | 1 | | | 3 | | | 1 | 4 | 3 | 3 |
| Abil^{®} EM 90 | | | | | | | 4 | | | | |
| Isolan^{®} PDI | | | | | | 4 | | | | | |
| Glucate^{®} DO | | | | 5 | | | | | | | |
| Arlacel^{®} 83 | | | 5 | | | | | | | | |
| Dehymuls^{®} FCE | | | | | | | | | | | |
| Dehymuls^{®} HRE 7 | | | | | | | | 4 | | 1 | |
| Zinkstearat | 2 | 1 | | 1 | 1 | | | 1 | 1 | 1 | |
| Mikrokristallines Wachs | | | 5 | | | 2 | | | | | 5 |
| Bienenwachs | 4 | | | 1 | | | | 1 | 4 | 7 | |
| Emulgade^{®} SUCRO | 2 | 0.5 | | 1 | 1 | | 1 | | | | 1 |
| Tego Care^{®} CG | | | | | 1 | | | | | | 0.5 |
| Prisorine^{®} 3505 | | | 1 | 1 | | 1 | 1 | | | | 1 |
| Dry Flo^{®} Plus | | | | | | | | | | | |
| SFE 839 | | | | | | | 3 | | | | |
| Emery^{®} 1780 | 1 | | | | | | | | | | 1 |
| Lanolin; anhydrous USP | | | 5 | | | | | | | 4 | |
| **erfindungsgemäße Ölkomponente** | **3** | **4** | **2** | **12** | **10** | **2** | **2** | **6** | **3** | **12** | **1** |
| Cegesoft^{®} C 17 | | | 3 | | | | | | | 1 | |
| Myritol^{®} PC | | | | | | 2 | | 4 | | | |
| Myritol^{®} 331 | 6 | | | | 2 | 6 | 2 | | | | 8 |
| Finsolv^{®} TN | | | | 5 | | 2 | 5 | | | | |
| Cetiol^{®} A | | 6 | | | | 4 | | | | | |
| Cetiol^{®} CC | | 8 | | | 2 | 2 | 2 | | | | 5 |
| Cetiol^{®} SN | | 5 | | | | | | 3 | | | |
| Cetiol^{®} OE | 3 | | | | 4 | | 2 | | 4 | 2 | |
| Dow Coming DC^{®} 244 | | | | | 1 | | 2 | | | | |
| Dow Coming DC^{®} 2502 | | | 1 | | 2 | | | | | | |
| Prisorine^{®} 3758 | | | | | 3 | | | | | | |
| Silikonöl Wacker AK^{®} 350 | | | | 4 | | | | 3 | | | |
| Cetiol^{®} 868 | | | | | | | | | | 2 | 7 |
| Cetiol^{®} J 600 | | | 4 | | | 2 | | | | | |
| Ceraphyl^{®} 45 | | | | 2 | | | | 2 | | 6 | |
| Mineralöl | | | | | 4 | | | | | | |
| Cetiol^{®} B | | | 2 | 4 | | | | | | 3 | |
| Eutanol^{®} G 16 | | 1 | | | | | | | | 3 | |
| Eutanol^{®} G | | | 3 | | | | | 8 | | | |
| Cetiol^{®} PGL | | | | | | 4 | | | 9 | | |
| Mandelöl | | | | | 1 | | 5 | | | | |
| Insect Repellent^{®} 3535 | 2 | | | | | | | | | | |
| N,N-Diethyl-m-toluamid | | | | 3 | | | | 5 | | | |
| Photonyl^{®} LS | 2 | 2 | | | | | | | | | |
| Panthenol | 1.0 | | | | | | | | | | |
| Bisabolol | 0.2 | | | | | | | | | | |
| Tocopherol / Tocopheryl Acetate | 1.0 | | | | | | | | | | |
| Magnesiumsulfat x 7 Wasser | 1 | | | | | | | | | | |
| Bentone^{®} 38 | | | | | 1 | | | | | | |
| Propylencarbonat | | | | | 0.5 | | | | | | |
| Ethanol | | | | | | | | | | 8 | |
| Butylene Glycol | | | 2 | 6 | | | 2 | 5 | | | 2 |
| Glycerin | 5 | 3 | 3 | | 5 | 3 | 2 | | 10 | 4 | |
| Wasser, Konservierungsmittel | Ad 100, q.s. | | | | | | | | | | |

| **Anhang** | | | |
|---|---|---|---|
| 1) | Abil^{®} EM 90 INCI: Cetyl Dimethicone Copolyol Hersteller: Tego Cosmetics (Goldschmidt) | 13) | Cegesoft^{®} C 17 INCI: Myristyl Lactate Hersteller: Cognis Deutschland GmbH, Grünau |
| 2) | Allianz^{®} OPT INCI: AcrylateslC12-22 Alkyl Methacrylate Copolymer Hersteller: Rohm und Haas | 14) | Cegesoft^{®} PFO INCI: Passiflora Incamata (EU) Hersteller: Cognis Deutschland GmbH |
| 3) | Amphisol^{®} K INCI: Potassium Cetyl Phosphate Hersteller: Hoffmann La Roche | 15) | Cegesoft^{®} PS 6 INCI: Olus Hersteller: Cognis Deutschland GmbH |
| 4) | Antaron^{®} V 220 INCI: PVP/Eicosene Copolymer Hersteller: GAF General Aniline Firm Corp. (IPS-Global) | 16) | Ceraphyl^{®} 45 INCI: Diethylhexyl Malate Hersteller: International Specialty Products |
| 5) | Antaron^{®} V 216 INCI: PVP/Hexadecene Copolymer Hersteller: GAF General Aniline Firm Corp. (IPS-Global) | 17) | Cetiol^{®} 868 INCI: Ethylhexyl Stearate Hersteller: Cognis Deutschland GmbH |
| 6) | Arlacel^{®} 83 INCI: Sorbitan Sesquioleate Hersteller: Uniqema (ICI Surfacants) | 18) | Cetiol^{®} A INCI: Hexyl Laurate Hersteller: Cognis Deutschland GmbH |
| 7) | Arlacel^{®} P 135 INCI: PEG-30 Dipolyhydroxystearate Hersteller: Uniqema (ICI Surfacants) | 19) | Cetiol^{®} B INCI: Dibutyl Adipate Hersteller: Cognis Deutschland GmbH |
| 8) | Bentone^{®} 38 INCI: Quaternium-18 Hectorite Hersteller: Rheox (Elementis Specialties) | 20) | Cetiol^{®} CC INCI: Dicaprylyl Carbonate Hersteller: Cognis Deutschland GmbH |
| 9) | Carbopol^{®} 980 INCI: Carbomer Hersteller: Goodrich | 21) | Cetiol^{®} J 600 INCI: Oleyl Erucate Hersteller: Cognis Deutschland GmbH |
| 10) | Carbopol^{®} 2984 INCI: Carbomer Hersteller: Noveon, Inc. | 22) | Cetiol^{®} LC INCI: Coco-Caprylate/Caprate Hersteller: Cognis Deutschland GmbH |
| 11) | Carbopol^{®} ETD 2001 INCI: Carbomer Hersteller: Noveon, Inc. | 23) | Cetiol^{®} OE INCI: Dicaprylyl Ether Hersteller: Cognis Deutschland GmbH |
| 12) | Carbopol^{®} Ultrez 10 INCI: Carbomer Hersteller: Noveon, Inc. INCI: Shea Butter Butyrospermum Parkii (Linne) Hersteller: Cognis Deutschland GmbH | 24) | Cetiol^{®} PGL INCI: Hexyldecanol, Hexyldecyl Laurate Hersteller: Cognis Deutschland GmbH |
| | | 25) | Cetiol^{®} S INCI: Diethylhexylcyclohexane Hersteller: Cognis Deutschland GmbH |
| | | 26) | Cetiol^{®}SB 45 INCI: Shea Butter Butyrospermun Parkii (Linne) Hersteller: Cognis Deutschland GmbH |
| 27) | Cetiol^{®} SN INCI: Cetearyl Isononanoate Hersteller: Cognis Deutschland GmbH | 39) | Dow Corning^{®} 244 Fluid INCI: Cyclomethicone Hersteller: Dow Coming |
| 28) | Copherol^{®} F 1300 C INCI: Tocopherol Hersteller: Cognis Deutschland GmbH | 40) | Dow Corning^{®} 246 Fluid INCI: Cyclopentasiloxane Hersteller: Dow Coming |
| 29) | Copherol 1250 C INCI: Tocopheryl Acetate Hersteller: Cognis Deutschland GmbH | 41) | Dow Corning^{®}2502 INCI: Cetyl Dimethicone Hersteller: Dow Coming |
| 30) | Cosmedia^{®} DC INCI: Hydrogenated Dimer Dilinoleyl / Dimethylcarbonate Copolymer Hersteller: Cognis Deutschland GmbH | 42) | Dry^{®}Flo Plus INCI: Aluminium Starch Octenylsuccinate Hersteller: National Starch |
| 31) | Cosmedia^{®} SP INCI: Sodium Polyacrylate Hersteller: Cognis Deutschland GmbH | 43) | Elfacos^{®}ST 37 INCI: PEG-22 Dodecyl Glycol Copolymer Hersteller: Akzo-Nobel |
| 32) | Cutina^{®} E 24 INCI: PEG-20 Glyceryl Stearate Hersteller: Cognis Deutschland GmbH | 44) | Elfacos^{®}ST 9 INCI: PEG-45 Dodecyl Glycol Copolymer Hersteller: Akzo-Nobel |
| 33) | Cutina^{®} HR INCI: Hydrogenated Castor Oil Hersteller: Cognis Deutschland GmbH | 45) | Emery^{®} 1780 INCI: Lanolin Alcohol Hersteller: Cognis Corporation (Emery) |
| 34) | Cutina^{®} MD INCI: Glyceryl Stearate Hersteller: Cognis Deutschland GmbH | 46) | Emulgade^{®} CM INCI: Cetearyl Isononanoate and Ceteareth-20 and Cetearyl Alcohol and Glyceryl Stearate and Glycerin and Ceteareth-12 and Cetyl Palmitate Hersteller: Cognis Deutschland GmbH |
| 35) | Cuitina^{®} PES INCI: Pentaerythrityl Distearate Hersteller: Cognis Deutschland GmbH | 47) | Emulgade^{®}PL 68/50 INCI: Cetearyl Glucoside, Cetearyl Alcohol Hersteller: Cognis Deutschland GmbH |
| 36) | Dehymuls^{®} FCE INCI: Dicocoyl Pentaerythrityl Distearyl Citrate Hersteller: Cognis Deutschland GmbH | 48) | Emulgade^{®} SE - PF INCI: Glyceryl Stearate (and) Ceteareth-20 (and) Ceteareth-12 (and) Cetearyl Alcohol (and) Cetyl Palmitate Hersteller: Cognis Deutschland GmbH |
| 37) | Dehymuls^{®} HRE 7 INCI: PEG-7 Hydrogenated Castor Oil Hersteller: Cognis Deutschland GmbH | 49) | Emulgade^{®} SUCRO INCI: Sucrose Polystearate (and) Hydrogenated Polyisobutene Hersteller: Cognis Deutschland GmbH |
| 38) | Dehymuls^{®} PGPH INCI: Polyglyceryl-2 Dipolyhydroxystearate Hersteller: Cognis Deutschland GmbH INCI: Ceteareth- 20 Hersteller: Cognis Deutschland GmbH | 50) | Eumulgin^{®} B1 INCI: Ceteareth-12 Hersteller: Cognis Deutschland G mbH |
| | | 51) | Eumulgin^{®} B 2 Hersteller: NRC Nordmann/Rassmann |
| 52) | Eumulgin^{®} HRE 40 INCI: PEG-40 Hydrogenated Castor Oil Hersteller: Cognis Deutschland GmbH | 64) | Hispagel^{®} 200 INCI: Glycerin, Glyceryl Polyacrylate Hersteller: Cognis Deutschland GmbH |
| 53) | Eumulgin^{®} SG INCI: Sodium Stearoyl Glutamate Hersteller: Cognis Deutschland GmbH | 65) | Hostaphat^{®} KL 340 N INCI: Tritaureth-4 Phosphate Hersteller: Clariant |
| 54) | Eumulgin^{®} VL 75 INCI: Lauryl Glucoside (and) Polyglyceryl-2 Di-polyhydroxystearate (and) Glycerin Hersteller: Cognis Deutschland GmbH | 66) | Hydagen^{®} C.A.T. INCI Triethyl Citrate Hersteller: Cognis Deutschland GmbH |
| 55) | Eusolex^{®} OCR INCI: Octocrylene Hersteller: Merck | 67) | Hydagen^{®}DCMF INCI: Chitosan Hersteller: Cognis Deutschland GmbH |
| 56) | Eusolex^{®} T 2000 INCI: Titanium Dioxide, Alumina, Simethicone Hersteller: Merck | 68) | Insect Repellent^{®} 3535 INCI : Ethyl Butylacetylaminopropionate Hersteller : EMD Chemicals Inc |
| 57) | Eusolex^{®} T AQUA INCI: Water and Titanium Dioxide and Alumina and Sodium Metaphosphate and Phenoxyethanol and Sodium Methylparaben Hersteller: Merck | 69) | Isolan^{®} PDI INCI: Diisostearoyl Polyglyceryl-3 Diisostearate Hersteller: Goldschmidt AG |
| 58) | Eutanol^{®} G INCI: Octyldodecanol Hersteller: Cognis Deutschland GmbH | 70) | Keltrol^{®} T INCI: Xanthan Gum Hersteller: CP Kelco |
| 59) | Eutanol^{®}G 16 INCI: Hexyldecanol Hersteller: Cognis Deutschland GmbH | 71) | Lameform^{®} TGI INCI: Polyglyceryl-3 Diisostearate Hersteller: Cognis Deutschland GmbH |
| 60) | Eutanol^{®}G 16 S INCI: Hexyldecyl Stearate Hersteller: Cognis Deutschland GmbH | 72) | Lanette^{®} 14 INCI: Myristyl Alcohol Hersteller: Cognis Deutschland GmbH |
| 61) | Finsolv^{®} TN INCI: C 12/15 Alkyl Benzoate Hersteller: Findex (Nordmann/Rassmann) | 73) | Lanette 18 INCI: Stearyl Alcohol Hersteller: Cognis Deutschland GmbH |
| 62) | Generol^{®} R INCI: Brassica Campestris (Rapseed) Sterols Hersteller: Cognis Deutschland GmbH | 74) | Lanette^{®} 22 INCI: Behenyl Alcohol Hersteller: Cognis Deutschland GmbH |
| 63) | Glucate^{®} DO INCI: Methyl Glucose Dioleate INCI: Aluminium Chlorhydrate Hersteller: Clariant | 75) | Lanette^{®} E INCI: Sodium Cetearyl Sulfate Hersteller: Cognis Deutschland GmbH |
| | | 76) | Lanette^{®} O INCI: Cetearyl Alcohol Hersteller: Cognis Deutschland GmbH |
| | | 77) | Locron^{®} L Hersteller: Symrise |
| 78) | Lucentite^{®} SAN INCI: Quaternium-18 Hectoritr Hersteller: Co-Op Chemical Co., Ltd. | 83) | Neo Heliopan^{®} OS INCI: Ethylhexyl Salicylate Hersteller: Symrise |
| 79) | Monomuls^{®} 90-018 INCI: Glyceryl Oleate Hersteller: Cognis Deutschland GmbH | 84) | Novata^{®} AB INCI: Cocoglycerides Hersteller: Cognis Deutschland GmbH |
| 80) | Myrj^{®} 51 INCI: PEG-30-Sterate Hersteller: Uniqema | 85) | Parsol^{®} 1789 INCI: Butyl Methoxydibenzoylmethane Hersteller: Hoffmann-La Roche (Givaudan) |
| 81) | Myritol^{®} 312 INCI: Caprylic/Capric Triglyceride Hersteller: Cognis Deutschland GmbH | 86) | Pemulen^{®} TR-2 Polymer INCI: Acrylates / C10-30 Alkylacrylate Cross-polymer Hersteller: Noveon, Inc. |
| 82) | Myritol^{®} 331 INCI: Cocoglycerides Hersteller: Cognis Deutschland GmbH | 87) | Photonyl^{®} LS INCI: Arginine, Disodium Adenosine Triphosphate, Mannitol, Pyridoxine HCL, Phenylalanine, Tyrosine Hersteller: Laboratoires Serobiologiques (Cognis) |
| 83) | Myritol^{®} PC INCI: Propylene Glycol Dicaprylate/Dicaprate Hersteller: Cognis Deutschland GmbH | 88) | Prisorine^{®} 3505 INCI: Isostearic Acid Hersteller: Uniqema |
| 84) | Neo Heliopan^{®} 303 INCI: Octocrylene Hersteller: Symrise | 89) | Prisorine^{®} 3758 INCI: Hydrogenated Polyisobutene Hersteller: Uniqema |
| 85) | Neo Heliopan^{®} AP INCI: Disodium Phenyl Dibenzimidazole Tetrasulfonate Hersteller: Symrise | 90) | Rezal 36G INCI: Aluminum Zirconium Tetrachlorohydrex GLY Hersteller: Reheis, Inc |
| 86) | Neo Heliopa^{®} AV INCI: Ethylhexyl Methoxycinnamate Hersteller: Symrise | 91) | SFE^{®} 839 INCI: Cyclopentasiloxane and Dimethicone/Vinyl Dimethicone Crosspolymer Hersteller: GE Silicones |
| 87) | Neo Heliopan^{®} BB INCI: Benzophenone-3 Hersteller: Symrise | 92) | Silikonöl Wacker AK^{®} 350 INCI: Dimethicone Hersteller: Wacker |
| 88) | Neo Heliopan^{®} E 1000 INCI: Isoamyl-p-Methoxycinnamate Hersteller: Symrise | 93) | Tego^{®} Care 450 INCI: Polyglyceryl-3 Methylglucose Distearate Hersteller: Tego Cosmetics (Goldschmidt) |
| 81) | Neo Heliopan^{®} Hydro INCI: Phenylbenzimidazole Sulfonic Acid Hersteller: Symrise | | |
| 82) | Neo Heliopan^{®} MBC INCI: 4-Methylbenzylidene Camphor | | |
| 94) | Tego^{®} Care CG 90 INCI: Cetearyl Glucoside Hersteller: Goldschmidt | 106) | Zinc Oxide NDM INCI: Zinc Oxide Hersteler: Symrise |
| 95) | Tegosoft^{®} DEC INCI: Diethylhexyl Carbonate Hersteller: Goldschmidt | | |
| 96) | Tinosorb^{®} S INCI: Bis-Ethylhexyloxyphenol Methoxyphenyl Triazine Hersteller: Ciba Specialty Chemicals Corporation | | |
| 97) | Tinosorb^{®} M INCI: Methylene Bis-Benzotriazolyl Tetramethylbutylphenol Herstelller: Ciba Specialty Chemicals Corporation | | |
| 98) | Tween^{®} 60 INCI: Polysorbate 60 Hersteller: Uniqema (ICI Surfactants) | | |
| 99) | Uvasorb^{®} HEB INCI: Diethylhexyl Butamido Triazone Hersteller: 3V Inc. | | |
| 100) | Unirep^{®} U-18 INCI: Dimethyl Phthalate and Diethyl Toluamide and Ethyl Hexanediol Hersteller: Induchem AG | | |
| 101) | Uvinul^{®} T 150 INCI: Ethylhexyl Triazone Hersteller: BASF | | |
| 102) | Uvinul^{®} A plus INCI: Diethylamino Hydroxybenzoyl Hexyl Benzoate Hersteller: BASF | | |
| 103) | Veegum^{®} Ultra INCI: Magnesium Aluminium Silicate Hersteller: R. T. Vanderbilt Company, Inc | | |
| 104) | Veegum^{®} Plus INCI: Magnesium Aluminum Silicate and Cellulose Gum Hersteller: R. T. Vanderbilt Company, Inc | | |
| 105) | Z-Cote^{®} HP 1 INCI: Zinc Oxide and Triethoxycaprylylsilane Hersteller: BASF | | |

## Patentansprüche

1. Alkylbenzoat Gemisch, **dadurch gekennzeichnet, dass** der Anteil an ungeradzahligen Alkylbenzoaten kleiner gleich 40%, insbesondere kleiner gleich 30%, bezogen auf die Gesamtsumme der Alkylbenzoate, beträgt.

2. Alkylbenzoat Gemisch, wobei die Summe der C12- und C14-Alkylbenzoate größer gleich 85%, bezogen auf die Gesamtsumme der Alkylbenzoate, beträgt.

3. Alkylbenzoat Gemisch nach Anspruch 2, **dadurch gekennzeichnet, dass** die Summe der C12-und C14-Alkylbenzoate größer gleich 90%, insbesondere größer gleich 95%, bezogen auf die Gesamtsumme der Alkylbenzoate, beträgt.

4. Alkylbenzoat Gemisch, enthaltend C12 und/oder C14 Alkylbenzoate wobei die Summe der Alkylbenzoate mit einer C-Kettenlänge von größer 14, kleiner gleich 15%, bezogen auf die Gesamtsumme der Alkylbenzoate, beträgt.

5. Alkylbenzoat Gemisch nach Anspruch 4, wobei die Summe der Alkylbenzoate mit einer C-Kettenlänge von größer 14, kleiner gleich 10%, insbesondere kleiner gleich 8 %, bevorzugt kleiner gleich 4%, insbesondere kleiner gleich 2% beträgt.

6. Alkylbenzoat Gemisch, wobei die Alkylbenzoate folgende C-Kettenverteilung aufweisen
(a) C-12 Alkylbenzoate größer gleich 60%, bevorzugt größer gleich 65%, insbesondere größer gleich 70 % und
(b) C-14 Alkylbenzoate zwischen 15% und 40%, insbesondere zwischen 20% und 35%, bevorzugt zwischen 25% und 30%.
bezogen auf die Gesamtsumme der Alkylbenzoate.

7. Alkylbenzoat Gemisch nach einem der Ansprüche 1 bis 6, **dadurch gekennzeichnet, dass** die Summe der Alkylbenzoate mit einer C-Kettenlänge von kleiner gleich 10, kleiner gleich 3%, insbesondere kleiner gleich 2%, bevorzugt kleiner gleich 1,5%, insbesondere kleiner gleich 1 %, bezogen auf die Gesamtsumme der Alkylbenzoate, beträgt.

8. Alkylbenzoat Gemisch nach einem der Ansprüche 1 bis 7, **dadurch gekennzeichnet, dass** Summe der Alkylbenzoate mit einer C-Kettenlänge von größer 14, kleiner gleich 15%, insbesondere kleiner gleich 10%, insbesondere kleiner gleich 8%, bevorzugt kleiner gleich 4%, bezogen auf die Gesamtsumme der Alkylbenzoate, beträgt.

9. Alkylbenzoate Gemisch nach einem der Ansprüche 1 bis 8, **dadurch gekennzeichnet, dass** die Summe der verzweigten Alkylbenzoate kleiner gleich 10 %, bevorzugt kleiner gleich 5 %, bezogen auf die Gesamtsumme der Alkylbenzoate, beträgt.

10. Kosmetische und/oder pharmazeutische Zubereitungen, enthaltend mindestens ein Alkylbenzoat Gemisch gemäß einem der Ansprüche 1 bis 9 und mindestens einen UV-Lichtschutzfilter.

11. Verwendung der Alkylbenzoate nach einem der Ansprüche 1 bis 9 in kosmetischen und/oder pharmazeutischen Zubereitungen.

12. Verwendung nach Anspruch 11 als Ölkomponente.
